# EUROPEAN PATENT SPECIFICATION

(11) **EP 3 011 333 B1**
(45) Date of publication and mention of the grant of the patent: **08.09.2021**
(21) Application number: 14737010.0
(22) Date of filing: 20.06.2014
(51) Int. Cl.: G01N 33/50

(54) **MARKERS FOR LONG-TERM KIDNEY GRAFT DYSFUNCTION**
MARKER VON LANGFRISTIGER NIERENTRANSPLANTATDYSFUNKTION
MARQUEURS POUR DYSFONCTION DE GREFFON RÉNAL À LONG TERME

(30) Priority: 21.06.2013 EP 13290148
(43) Date of publication of application: 27.04.2016
(73) Proprietor: Institut National de la Santé et de la Recherche Médicale, 75013 Paris (FR); Centre Hospitalier Universitaire De Nantes, 44000 Nantes (FR); Universite de Nantes, 44000 Nantes (FR)
(72) Inventor: DEGAUQUE, Nicolas, F-44000 Nantes (FR); BROUARD, Sophie, F-44000 Nantes (FR); GIRAL, Magali, F-44000 Nantes (FR); FOUCHER, Yohann, F-44000 Nantes (FR); YAP, Michelle, F-44000 Nantes (FR); SOULILLOU, Jean-Paul, F-44000 Nantes (FR)
(74) Representative: Nony
(86) International application number: PCT/IB2014/062469
(87) International publication number: WO 2014/203214

(56) References cited:
- BAETEN DOMINIQUE ET AL: "Phenotypically and functionally distinct CD8+ lymphocyte populations in long-term drug-free tolerance and chronic rejection in human kidney graft recipients.", JOURNAL OF THE AMERICAN SOCIETY OF NEPHROLOGY : JASN JAN 2006, vol. 17, no. 1, January 2006 (2006-01), pages 294-304, XP002713866, ISSN: 1046-6673
- BETJES MICHIEL G H ET AL: "Terminally differentiated CD8+ Temra cells are associated with the risk for acute kidney allograft rejection.", TRANSPLANTATION 15 JUL 2012, vol. 94, no. 1, 15 July 2012 (2012-07-15), pages 63-69, XP009173026, ISSN: 1534-6080
- D'ASARO M ET AL: "Increase of CCR7<-> CD45RA<+> CD8 T cells (TEMRA) in chronic graft-versus-host disease [15]", LEUKEMIA 200603 GB, vol. 20, no. 3, March 2006 (2006-03), pages 545-547, XP002713867, ISSN: 0887-6924
- D' ASARO M ET AL: "Analysis of memory and effector CD8+ T cell subsets in chronic graft-versus-host disease.", INTERNATIONAL JOURNAL OF IMMUNOPATHOLOGY AND PHARMACOLOGY 2009 JAN-MAR, vol. 22, no. 1, January 2009 (2009-01), pages 195-205, XP009173031, ISSN: 0394-6320
- CHATTOPADHYAY P K ET AL: "The cytolytic enzymes granyzme A, granzyme B, and perforin: Expression patterns, cell distribution, and their relationship to cell maturity and bright CD57 expression", JOURNAL OF LEUKOCYTE BIOLOGY 20090101 US, vol. 85, no. 1, 1 January 2009 (2009-01-01), pages 88-97, XP002713869, ISSN: 0741-5400 cited in the application
- GEGINAT J ET AL: "Proliferation and differentiation potential of human CD8+ memory T-cell subsets in response to antigen or homeostatic cytokines", BLOOD, AMERICAN SOCIETY OF HEMATOLOGY, US, vol. 101, no. 11, 6 February 2003 (2003-02-06), pages 4260-4266, XP002409702, ISSN: 0006-4971, DOI: 10.1182/BLOOD-2002-11-3577
- PATRICK MIQUEU ET AL: "Analysis of the peripheral T-cell repertoire in kidney transplant patients", EUROPEAN JOURNAL OF IMMUNOLOGY, vol. 40, no. 11, 27 November 2010 (2010-11-27), pages 3280-3290, XP055081344, ISSN: 0014-2980, DOI: 10.1002/eji.201040301
- None

## Description

### FIELD OF THE INVENTION

The invention relates to *in vitro* methods and kits for determining the risk of occurrence of long-term kidney graft dysfunction in a subject. Thus the invention also relates to methods for preventing long-term kidney graft dysfunction in a subject

### BACKGROUND OF THE INVENTION

Despite the effectiveness of immunosuppressive drugs, kidney transplants are still subject to dysfunction in the long term. The major immunological cause of late kidney graft failure is considered to be chronic antibody-mediated rejection, which will also be referred herein as CAMR (Colvin et al. Antibody-mediated organ-allograft rejection. Nat Rev Immunol. 2005;5(10):807-817).

Its diagnosis relies on renal dysfunction, histological features and serum donor-specific antibodies, also referred herein as DSA (Sis et al. Banff '09 Meeting Report: Antibody Mediated Graft Deterioration and Implementation of Banff Working Groups. Am J Transplant. 2010;10(3):464-471).

However, the biological mechanisms leading to CAMR are poorly defined.

Anti-donor antibodies have been identified as a negative prognostic parameter for graft survival (Hourmant et al. Frequency and Clinical Implications of Development of Donor-Specific and Non-Donor-Specific HLA Antibodies after Kidney Transplantation. Journal of the American Society of Nephrology. 2005;16(9):2804-2812; Soulillou et al. Association between treatment-resistant kidney-allograft rejection and post-transplant appearance of antibodies to donor B-lymphocyte alloantigens. Lancet. 1978;1(8060):354-356).

The incidence of CAMR in patients with DSA has been reported to be 9 fold higher than in patients without DSA (Lefaucheur et al. Clinical Relevance of Preformed HLA Donor-SpecificAntibodies in Kidney Transplantation. Am J Transplant. 2007;8(2):324-331) and non-DSA antibodies could also have an impact on renal allograft outcome.

The involvement of T cells in the process of kidney dysfunction has recently regained interest. Pre-existing T cell memory is associated with high incidence and increased severity of rejection episodes (Poggio ED. Alloreactivity in Renal Transplant Recipients with and without Chronic Allograft Nephropathy. Journal of the American Society of Nephrology. 2004; 15(7): 1952-1960). For instance, recipients prone for acute rejection had a higher precursor frequency of alloreactive CD8 T cells than non-rejectors (van de Berg et al. Characteristics of alloreactive T cells measured before renal transplantation. Clinical&Experimental Immunology. 2012;168(2):241-250).

Using an experimental model of CAMR (Ballet et al. Indirect CD4 +TH1 Response, Anti donor Antibodies and Diffuse C4d Graft Deposits in Long-Term Recipients Conditioned by Donor Antigens Priming. Am J Transplant. 2009;9(4):697-708), the inventors previously reported that a similar TCR Vβ selection of CD8 T cells can be identified in the blood and graft of recipients (Lair et al. Functional Compartmentalization Following Induction of Long-Term Graft Survival with Pregraft Donor-Specific Transfusion. Am J Transplant. 2007;7(3):538-549).

CD8⁺ lymphocytes populations have been studied in different populations of patients, in particular in long-term drug-free tolerant patients, and in patients facing chronic-antibody mediated rejection of their graft (Baeten et al., Phenotypically and functionally distinct CD8+ lymphocyte populations in long-term drug-free tolerance and chronic rejection in human kidney graft recipients. J Am Soc Nephrol. 2006;17(1):294-304). CD8⁺ TEMRA cells populations have also been observed in patients before kidney transplantation (Betjes et al., Terminally differentiated CD8+ Temra cells are associated with the risk for acute kidney allograft rejection. Transplantation. 2012 Jul 15;94(1):63-9).

Collectively, the observations suggest that biases in the TCR Vβ repertoire of CD8 T cells are associated with kidney dysfunction.

Although the characterization of the TCR Vβ repertoire in a patient is known in the Art (see for instance the TcLandscape® approach which requires transcriptome analysis), there is an urgent need for alternative and more direct methods for assessing a risk of occurrence of long-term kidney graft dysfunction in a subject.

In particular there remains a need to identify specific sub-groups of T cells which may be used as markers of the risk of occurrence of long-term kidney graft dysfunction in a subject.

The inventors recently reported that different shapes of TCR Vβ repertoire are identified in patients with stable graft function (>5 years post-transplantation) despite the use of stringent clinical criteria to constitute a homogeneous group of kidney recipients (Miqueu et al. Analysis of the peripheral T-cell repertoire in kidney transplant patients. Eur. J. Immunol. 2010;40(11):3280-3290).

Thus there remains a need for novel methods and kits for determining the risk of occurrence of long-term kidney graft dysfunction in a subject.

There also remains a need for methods for preventing long-term kidney graft dysfunction in a subject.

In particular, there remains a need for methods and kits which may be used routinely and/or with a high predictability value.

There is also a need for methods which may be directly applicable to widespread techniques such as flow cytometry, and more particularly FACS (also known as Fluorescence-Activated Cell Sorting flow cytometry).

It is an object of the invention to address those needs.

### SUMMARY OF THE INVENTION

The present inventors have identified a link between (i) the presence of, and/or the level of, a sub-group of TEMRA CD8+ T cells, which is termed "highly differentiated cytotoxic TEMRA CD8+ T cells" herein, having a phenotype CD45RA⁺/CD197⁻/CD27⁻ /CD28⁻ and (ii) the risk of developing a kidney graft dysfunction.

In other words, it has been shown herein that the expansion of highly differentiated cytotoxic TEMRA CD8+ T cells defines a population at-risk of long-term kidney graft dysfunction.

These results establish that the regulation of effector CD8 T cells differentiation is deficient in patients with higher risk of long-term kidney graft dysfunction.

Thus, monitoring the phenotype of circulating CD8+ T cells may improve the identification of such at-risk patients, from a population of kidney recipients currently under standard immunosuppressive treatment with a stable graft function.

In view of the above, this specification describes an *in vitro* method for determining the risk of occurrence of long-term kidney graft dysfunction in a subject comprising:
a) measuring the level of CD8⁺ T cells having a phenotype CD45RA⁺/CD197⁻ /CD27⁻/CD28⁻ in a blood sample of the subject,
b) comparing the level of CD8⁺ T cells having a phenotype CD45RA⁺/CD197⁻ /CD27⁻/CD28⁻ measured at step a) with one or more reference values of the level of CD8⁺ T cells having a phenotype CD45RA⁺/CD197⁻/CD27⁻/CD28⁻, and
c) determining the risk of occurrence of long-term kidney graft dysfunction in the said subject from the comparison performed at step b).

In view of the above, this invention relates to an *in vitro* method for determining the risk of occurrence of long-term kidney graft dysfunction in a subject, said subject being a kidney recipient with current stable graft function, comprising:
a) measuring the level of CD8⁺ T cells having a phenotype CD45RA⁺/CD197⁻ /CD27⁻/CD28⁻ in a blood sample of the subject,
b) comparing the level of CD8⁺ T cells having a phenotype CD45RA⁺/CD197⁻ /CD27⁻/CD28⁻ measured at step a) with one or more reference values of the level of CD8⁺ T cells having a phenotype CD45RA⁺/CD197⁻/CD27⁻/CD28⁻, and
c) determining the risk of occurrence of long-term kidney graft dysfunction in the said subject from the comparison performed at step b), wherein an increased level of CD8⁺ T cells having a phenotype CD45RA⁺/CD197⁻/CD27⁻/CD28⁻ is indicative of a risk of occurrence of long-term kidney graft dysfunction.

This specification also discloses a kit for determining the risk of occurrence of long-term kidney graft dysfunction in a subject comprising one or more reagents for detecting or quantifying CD45RA, CD197, CD27 and CD28 in CD8+ T cells.

This invention also concerns a use of a kit comprising one or more reagents for detecting or quantifying CD45RA, CD197, CD27 and CD28 in CD8+ T cells; in an *in vitro* method for determining the risk of occurrence of long-term kidney graft dysfunction in a subject, said subject being a kidney recipient with current stable graft function.

The present specification also discloses a method for preventing long-term kidney graft dysfunction in a subject comprising: a) performing an *in vitro* method for determining the risk of occurrence of long-term kidney graft dysfunction in a subject in a blood sample of the subject, whereby the risk of occurrence of long-term kidney graft dysfunction in the subject is determined, and b) administering to the subject a suitable therapeutic treatment.

The present invention also pertains to an immunosuppressive treatment for use in a method for preventing long-term kidney graft dysfunction in a subject; wherein the subject is a kidney recipient with current stable graft function, determined at risk for an occurence of long-term kidney graft dysfunction, as defined above.

### BRIEF DESCRIPTION OF THE FIGURES

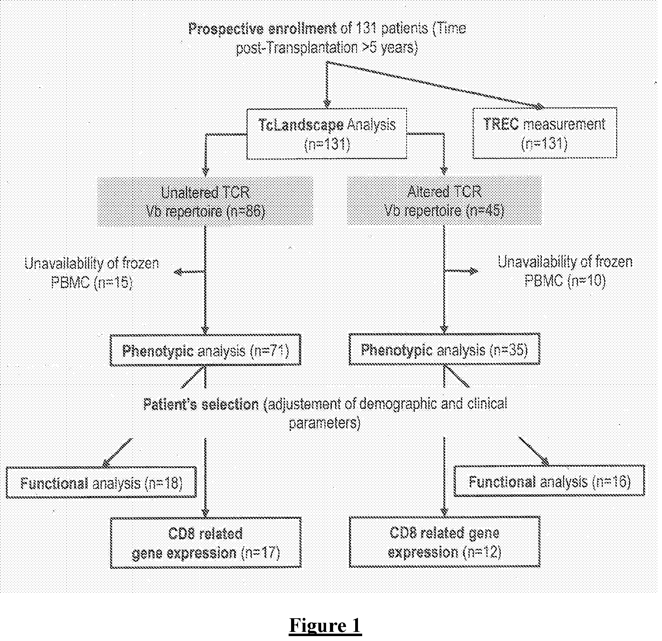

**Figure 1** **| Description of the observational and prospective study.** The number of patients is mentioned in the bracket.

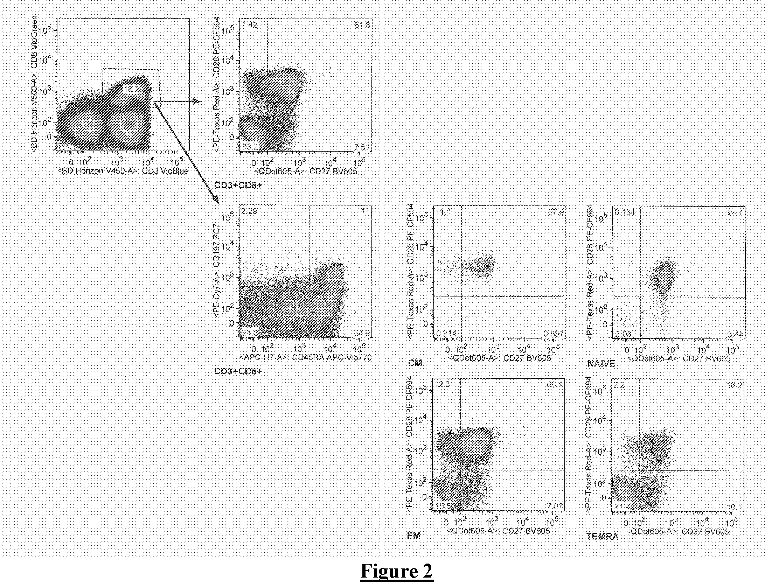

**Figure 2** **| Gating strategy for phenotypic characterization of CD8 T cells.** After isolating lymphocytes using a morphological gate (FSC vs. SSC) and viable lymphocytes by gating on Yellow negative cells, CD3⁺CD8⁺ cells were selected and subdivided into quadrants through CD27 & CD28 or CD45RA & CD197.

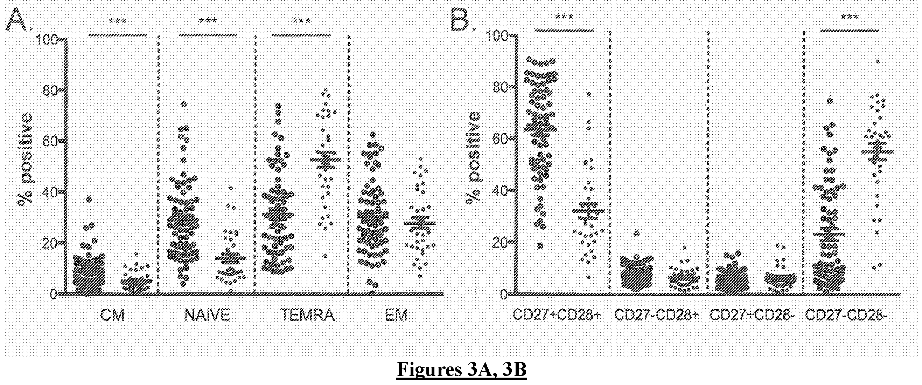

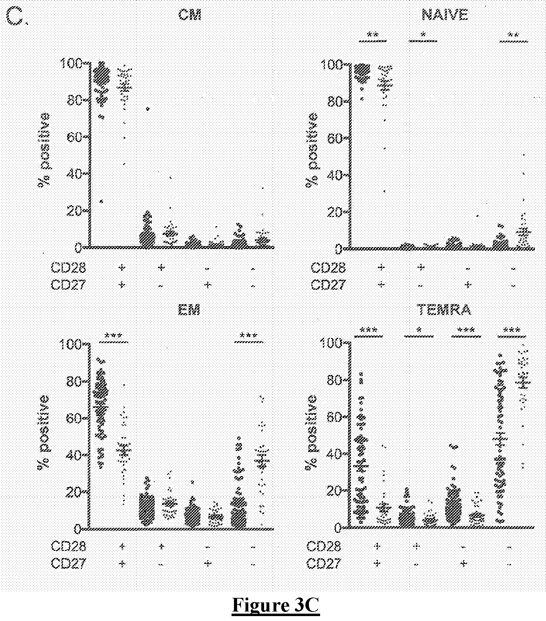

**Figure 3** | **Reduction in TCR Vβ repertoire diversity is associated with an increase of highly differentiated TEMRA (CD45RA⁺CD197⁻CD27⁻CD28⁻) CD8 effector T cells.** Expression of CD45RA and CD197 (A) and CD27 and CD28 (**B**) was measured in CD8 T cells in PBMC from STABLE patients with a diverse (n=71; open circle) or restricted TR Vβ repertoire (n=35; filled circle). CD3⁺CD8⁺ cells were gated by morphology and viability before being subdivided into (A) CM (Central Memory), Naive, TEMRA and EM (Effector Memory) subset based on the expression of CD45RA and CD197 or into (B) CD27 and CD28 subsets. (**C**) Expression of CD27 and CD28 within CM, Naïve, TEMRA and EM subsets. Each dot represents one individual, and the mean and the sem are displayed. Comparison of the frequency of the each CD8 subset was performed using a Holm multiple comparison test. *p< 0.05, ***p < 0.001).

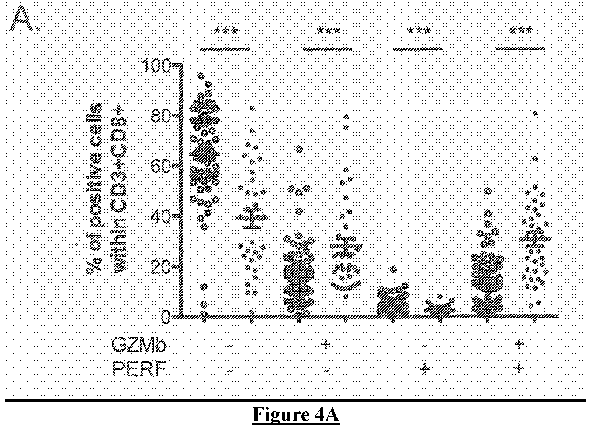

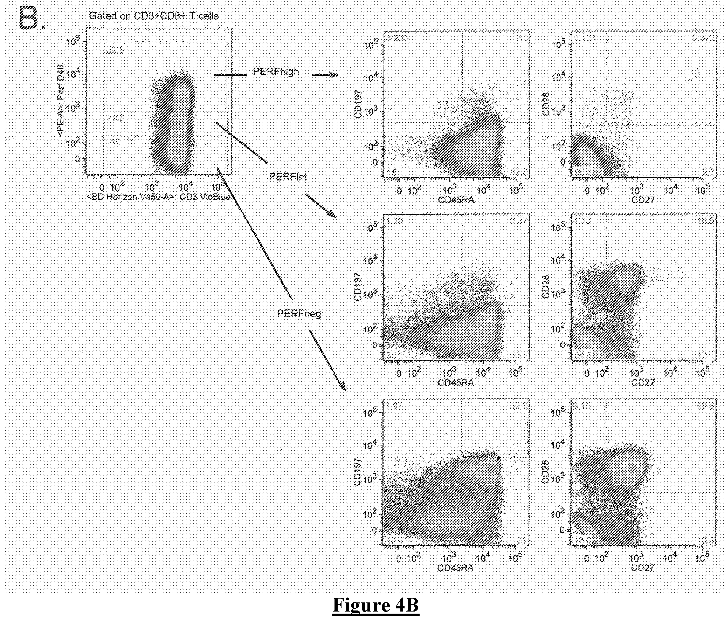

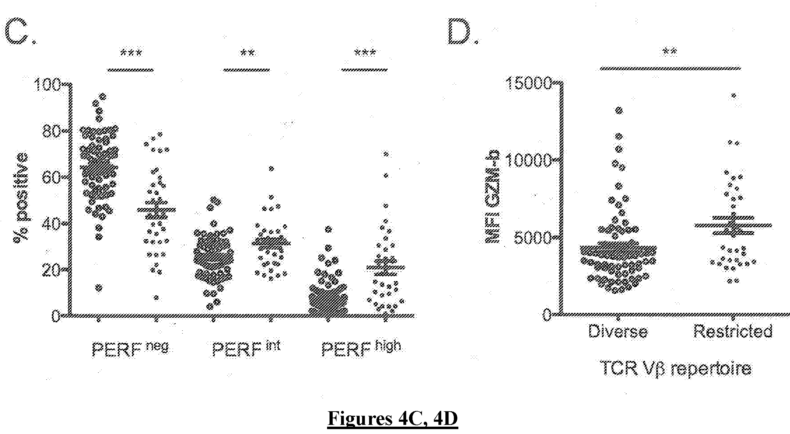

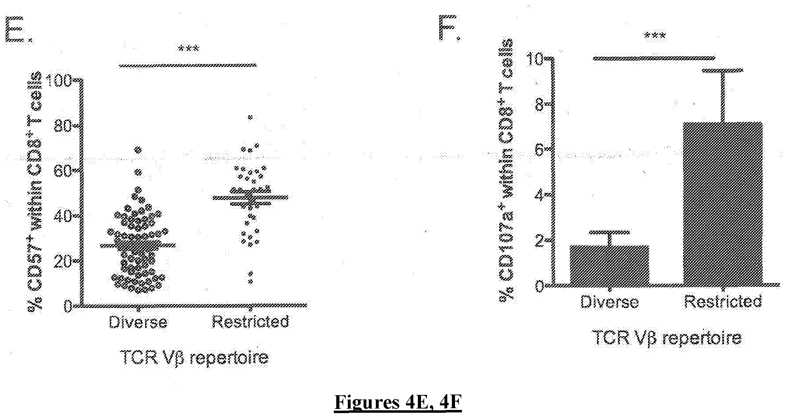

**Figure 4** **| CD8 T cells in patients with restricted TCR Vβ repertoire demonstrated a high expression of cytotoxic molecules (granzyme B and perforin) compared with patients with a polyclonal TCR Vβ repertoire. (A)** CD3⁺CD8⁺ cells from patients with a restricted TCR Vβ repertoire (n=35; filled circle) express high amount of GZM-b with and without the co-expression of perforin when compared to cells from diverse TCR Vβ repertoire patients(n=71; open circle). (**B**) Three levels of perforin expression could be identified. Representative flow cytometry plots showing CD45RA and CD197 or CD27 and CD28 by PERF^{high}, PERF^{int}, and PERF^{neg} CD3⁺CD8⁺ cells. (**C and E**) Frequency of PERF^{high}, PERF^{int}, and PERF^{neg} (**C**) and of CD57⁺ **(E)** CD3⁺CD8⁺ cells in patients with a diverse TCR Vβ repertoire (n=71; open circle) or with a restricted TCR Vβ repertoire (n=35; filled circle). (**D**) Level of expression of GZM-b (MFI) in patients with a diverse TCR Vβ repertoire (n=71; open circle) or with a restricted TCR Vβ repertoire (n=35; filled circle). (**F**) PBMC from patients with a diverse TCR Vβ repertoire (n=15) or with a restricted TCR Vβ repertoire (n=13) were stimulated with plate bound anti-CD3 anti-CD28.2 mAb for 6h and expression of CD107a was measured on CD8 T cells. Each dot represents one individual except for E, and the mean and the sem are displayed. Comparison of the frequency of the each CD8 subset was performed using a Holm multiple comparison test. **p< 0.01, ***p < 0.001.

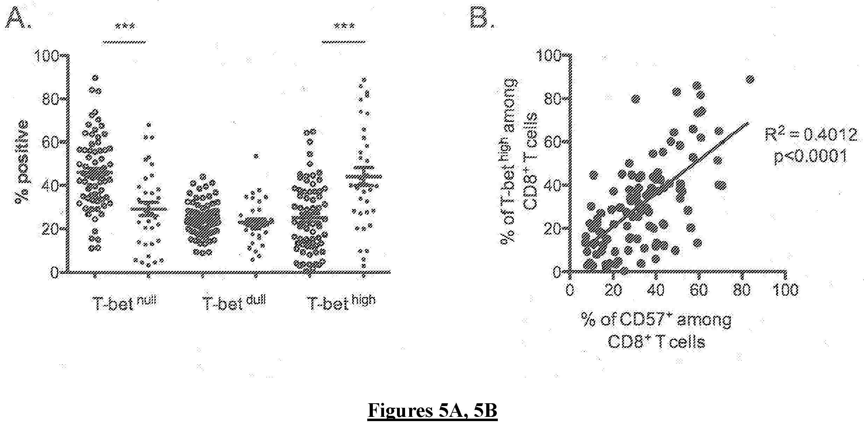

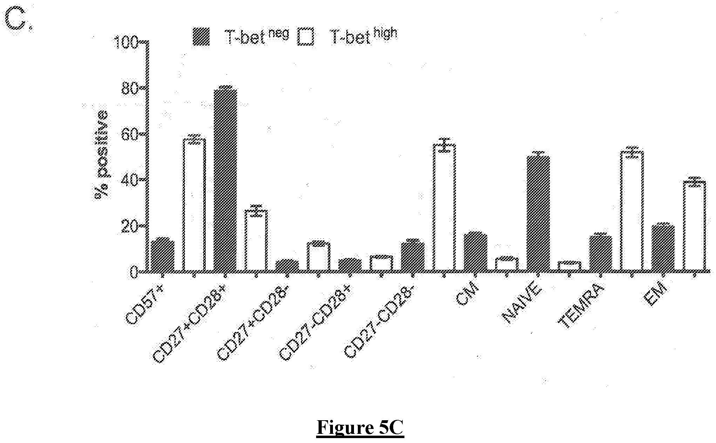

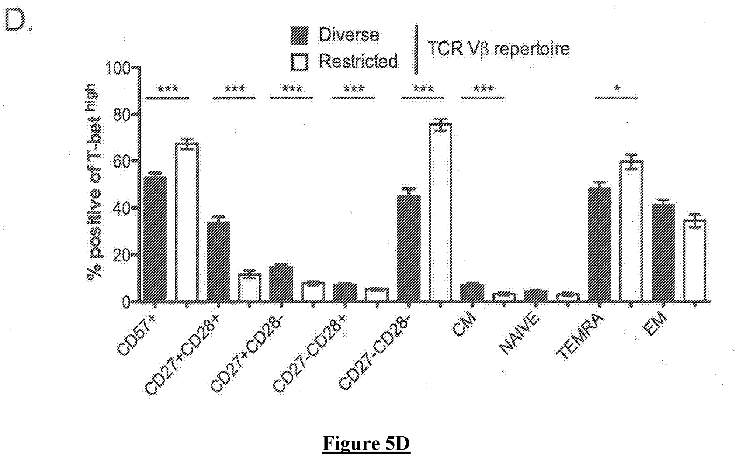

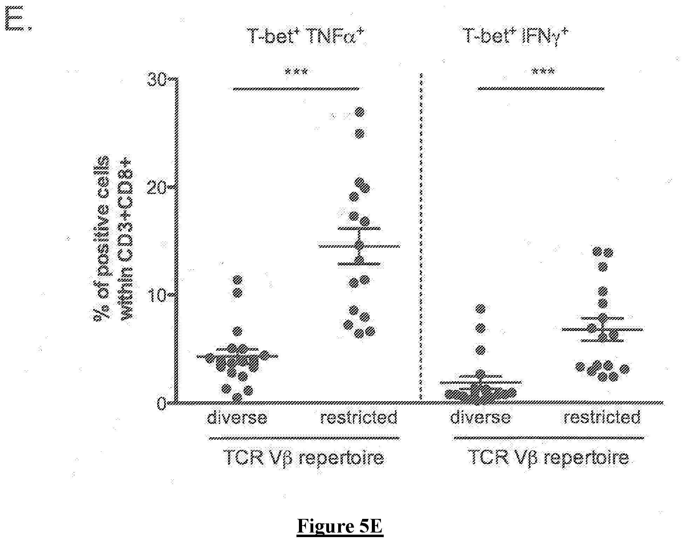

**Figure 5** **| CD8 T cells in patients with restricted TCR Vβ repertoire expressed higher levels of T-bet than patients with diverse TCR Vβ repertoire.** (A) Frequency of T-bet^{neg}, T-bet^{dull} and T-bet^{high} CD8 T cells was measured in CD8 T cells in PBMC from STABLE patients with a diverse (n=71; open circle) or restricted TR Vβ repertoire (n=35; filled circle). (**B**) The frequency of CD57⁺ CD8 T cells was plotted against the frequency of T-bet^{high} CD8 T cells within all patients. Linear regression was performed to determine statistical significance and regression factor is indicated. (**C**) Phenotype of T-bet^{neg} (white bar) versus T-bet^{high} (black bar) of the whole cohort. (**D**) Phenotype of T-bet^{high} CD8 T cells from STABLE patients with a diverse (n=71; black bar) or restricted TCR Vβ repertoire (n=35; white bar). (**E**) PBMC from patients with a diverse TCR Vβ repertoire (n=18) or with a restricted TCR Vβ repertoire (n=16) were stimulated with plate bound anti-CD3 anti-CD28.2 mAb for 6h and expression of T-bet, IFN-γ and TNF-α was measured on CD8 T cells. The mean and the sem are displayed. Comparison of the frequency of the each CD8 subset was performed using a Holm multiple comparison test. *p< 0.05, ***p < 0.001.

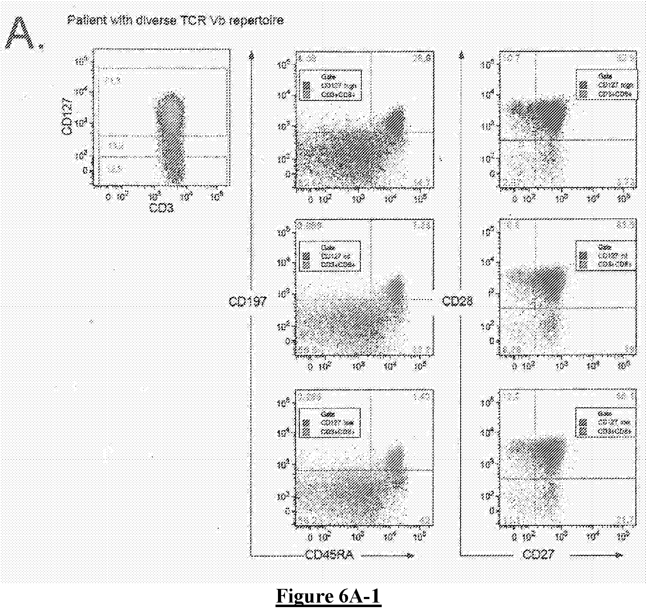

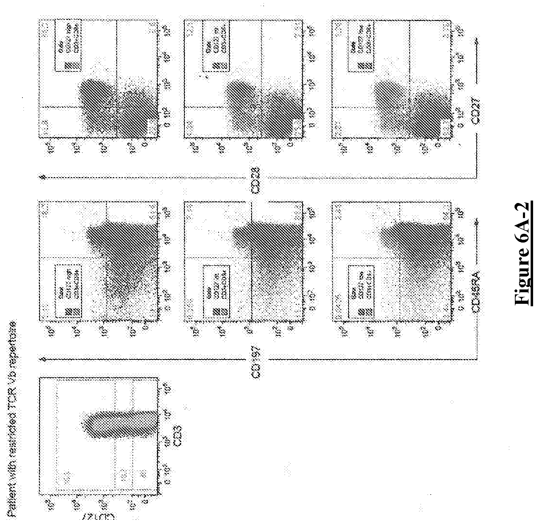

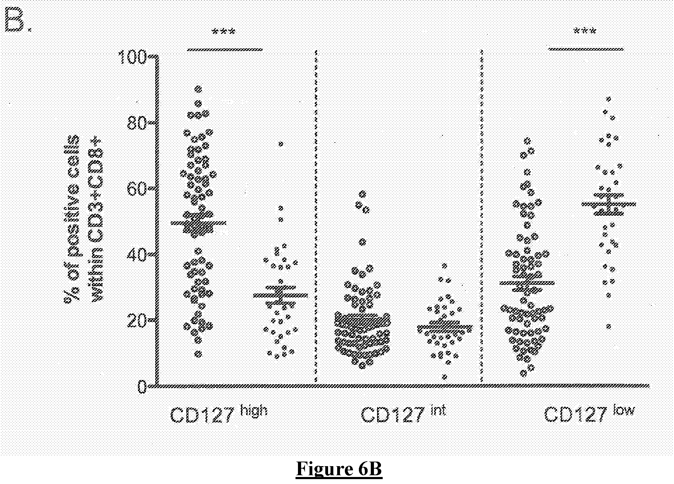

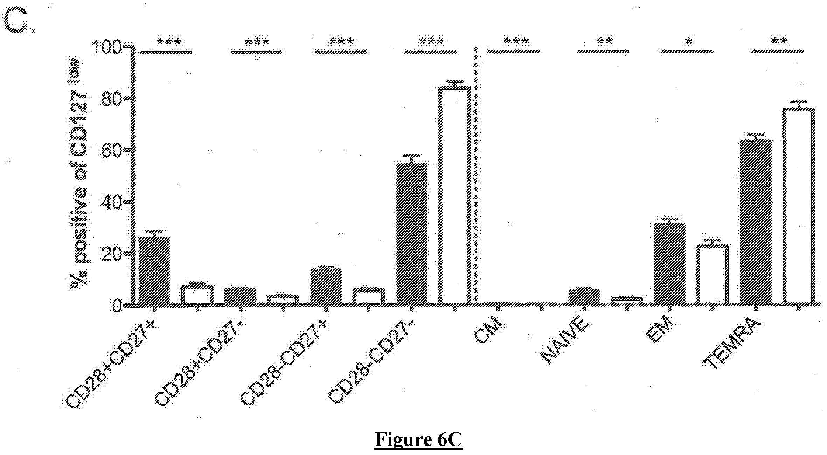

**Figure 6** **| Downregulation of CD127 by CD8 T cells in patients with restricted TCR Vβ repertoire.** (**A**) Three levels of CD127 expression could be identified. Representative flow cytometry plots showing CD45RA and CD197 or CD27 and CD28 by CD127^{high}, CD127^{int}, and CD127^{low} CD3⁺CD8⁺ cells in STABLE patients with a diverse (Figure 6A-1) or restricted TCR Vβ repertoire (Figure 6A-2). The various CD127 populations (blue) were overlaid onto dot plot (red) of total CD8⁺ T cells. (B) Frequency of CD127^{high}, CD127^{int} and CD127^{low}CD3⁺CD8⁺ cells in patients with a diverse TCR Vβ repertoire (n=71; open circle) or with a restricted TCR Vβ repertoire (n=35; filled circle). Each dot represents one individual, and the mean and the sem are displayed. (C) Phenotype of CD127^{low} CD8 T cells from STABLE patients with a diverse (n=71; black bar) or restricted TCR Vβ repertoire (n=35; white bar). The mean and the sem are displayed. Comparison of the frequency of the each CD8 subset was performed using a Holm multiple comparison test. *p< 0.05, ***p < 0.001.

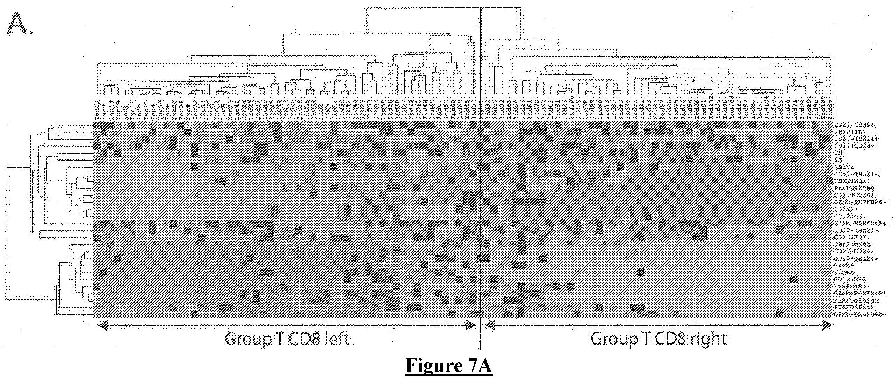

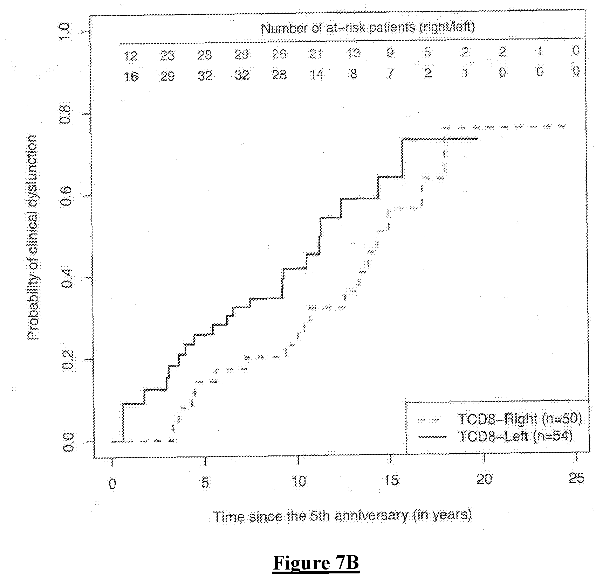

**Figure 7** **| Identification of patients at risk of late kidney dysfunction based on the features of CD8 T cells. (A)** Unsupervised clustering of kidney recipients based on CD8 T cells markers measured by flow cytometry at the time of inclusion. Two groups of patients (referred as left and right CD8 in B) are identified. **(B)** The cumulative probability of graft dysfunction was assessed in patients with stable graft function according to the CD8 based clustering ***(left vs. right CD8)*** and the time post-transplantation after the 5^{th} anniversary of transplantation. At the end of the follow-up, the low remaining number of transplant recipients explained the sudden increase in kidney dysfunction.

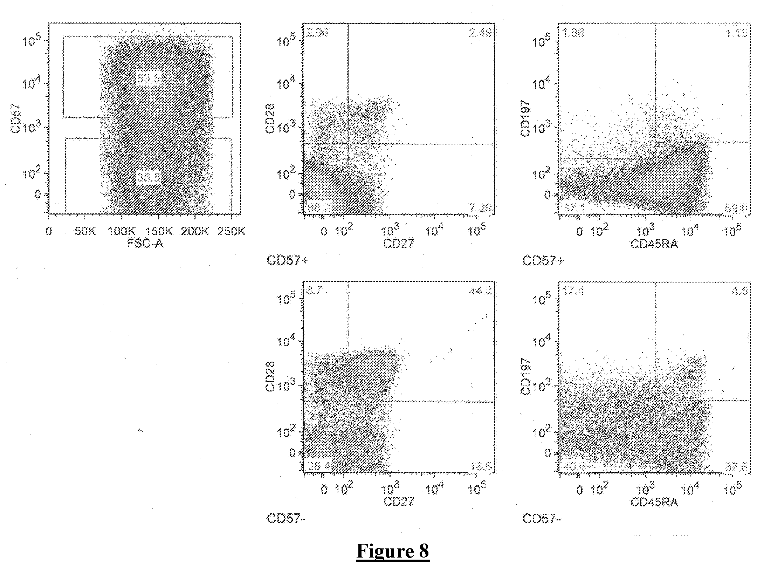

**Figure 8** **| Determination of the phenotype of CD57⁺ CD8 T cells** Representative flow cytometry plots. The results of Figure 8 show that CD57⁺ CD8 T cells were preferentially found within the CD45RA⁺CD197⁻CD27⁻CD28⁻ T cells.

### DETAILED DESCRIPTION OF THE INVENTION

It has been found according to the invention that the occurrence of an expansion of highly differentiated TEMRA CD8+ T cells in kidney-grafted individuals may be used as a sensitive, specific and reproducible marker of a risk of kidney dysfunction in those individuals.

In the art, TEMRA T cells are defined as terminally differentiated effector memory CD45RA+CD197⁻ T cells. More specifically, the highly differentiated TEMRA CD8+ T cells of the invention are CD8⁺ T cells having a phenotype CD45RA⁺/CD197⁻ /CD27⁻/CD28⁻.

They have shown that, in comparison to patients with a polyclonal TCR Vβ repertoire, CD8⁺ T cells from patients with a restricted TCR Vβ repertoire demonstrated a high expression of cytotoxic molecules (perforin and granzyme B) associated with high levels of T-bet expression that correlate with the expression of CD57 and the ability to secrete TNFα and IFNγ.

Despite exhibiting a stable graft function at the time of analysis, patients with reduced TCR Vβ repertoire diversity present an accumulation of highly differentiated TEMRA (CD45RA⁺CD197⁻CD27⁻CD28⁻) CD8⁺ T cells with all the attributes of cytotoxic and effector cells.

More specifically, the inventors have shown that reduction in thymic output does not account for the TCR Vβ repertoire alteration, and that an increase of highly differentiated TEMRA CD8⁺ T cells is a risk factor for kidney graft dysfunction.

What is more, using a multivariate regression model, the inventors have unexpectedly found that a 1.96 fold higher risk of long-term graft dysfunction was observed in patients with an increase of differentiated TEMRA CD8⁺ T cells at the inclusion.

With more than 7 additional years of clinical follow-up since the recruitment of the patients, the inventors report that patients with an increase of highly differentiated TEMRA CD8 T cells, based on the analysis of a plurality of markers including CD8, CD45RA, CD197 CD27 and CD28, individuals having the phenotype CD8⁺/CD45RA⁺/CD197⁻/CD27⁻/CD28⁻ have a higher risk of kidney graft dysfunction (hazard ratio of 1.96; p=0.0621).

Collectively, this study shows that the monitoring of CD8⁺ T cell differentiation and more particularly TEMRA CD8⁺ T cells of the invention is useful in the early stratification of patients into low and high risk of kidney dysfunction.

Further, the inventors have shown herein that an increase of highly differentiated TEMRA (CD45RA⁺CD197⁻CD27⁻CD28⁻) CD8⁺ T cells is correlated with the restriction of the TCR Vβ repertoire diversity. These further inventors findings confirm the accuracy of the statistical link that is disclosed herein between the presence of, and/or the increase of, highly differentiated TEMRA (CD45RA⁺CD197⁻CD27⁻CD28⁻) CD8⁺ T cells and the risk of kidney dysfunction in individuals who have undergone the grafting of a kidney or both kidneys.

Further, as it is shown herein, TEMRA CD8⁺ T cells also demonstrated a superior ability to express other markers, including CD57, perforin and granzyme B and over-expressed T-bet (transcription factor that drive effector function).

Still further, it is shown herein that T-bet^{high} CD8⁺ T cells were positively correlated with levels of CD57 as well as with the ability of these cells to secrete TNFα and IFNγ.

It is specified herein that the highly differentiated TEMRA CD8⁺ T cells are also CD3⁺ T cells.

According to the invention, TEMRA T cells refer to terminally differentiated effector memory CD45RA+CD197⁻ T cells.

More specifically, highly differentiated TEMRA CD8⁺ T cells of the invention are CD8⁺ T cells having a phenotype CD45RA⁺/CD197⁻/CD27⁻/CD28⁻.

Thus, the invention relates to the quantification of specific markers at the surface of CD8+ T cells within a biological sample.

This invention provides an *in vitro* method for determining the risk of occurrence of long-term kidney graft dysfunction in a subject comprising:
a) measuring the level of highly differentiated TEMRA CD8⁺ T cells in a blood sample of the subject,
b) comparing the level of highly differentiated TEMRA CD8⁺ T cells measured at step a) with one or more reference values of the level of highly differentiated TEMRA CD8⁺ T cells, and
c) determining the risk of occurrence of long-term kidney graft dysfunction in the said subject from the comparison performed at step b).

According to the invention, a "blood sample" is a biological sample which may encompass any blood sample, or derivative of a blood sample which comprises or which may comprise T cells.

A blood sample may be either a whole blood sample, or a sample which has been obtained after a step of fractionation.

In some embodiments of the said method, the quantification of the said markers is performed on a whole blood sample.

In some other embodiments of the said method, the quantification of markers is performed on a sample selected from the group consisting of (i) purified blood leukocytes, (ii) peripheral blood mononuclear cells or PBMC, (iii) purified lymphocytes, or (iv) purified CD8⁺ T cells.

According to an exemplary embodiment, the blood sample may be a sample consisting of peripheral blood mononuclear cells (PBMC).

As used herein, a "subject" encompasses subjects that were grafted with one or two syngeneic or non syngeneic kidney(s), including allogenic or even xenogenic, kidney(s). Said kidney transplanted subject may further have been grafted with another organ of the same donor providing the kidney, or a different donor.

In particular, the subject presents a well-functioning kidney graft and is under immunosuppressive treatment.

Kidney graft dysfunction encompasses graft dysfunction due to chronic antibody mediated rejection of a subject to his graft, as described in WO 2010142751. Graft dysfunction may be assessed on the basis of one or more physiological values selected in a group comprising (i) creatinemia above 25% of the basal level within the last 2 years of follow-up and (ii) a proteinuria >1g/24h. The group of patients studiedis composed of kidney recipients under standard immunosuppression with stable graft function.

This specification describes an *in vitro* method for determining the risk of occurrence of long-term kidney graft dysfunction in a subject comprising:
a) measuring the level of CD8⁺ T cells having a phenotype CD45RA⁺/CD197⁻ /CD27⁻/CD28⁻ in a blood sample of the subject,
b) comparing the level of CD8⁺ T cells having a phenotype CD45RA⁺/CD197⁻ /CD27⁻/CD28⁻ measured at step a) with one or more reference values of the level of CD8⁺ T cells having a phenotype CD45RA⁺/CD197⁻/CD27⁻/CD28⁻, and
c) determining the risk of occurrence of long-term kidney graft dysfunction in the said subject from the comparison performed at step b).

This specification describes an *in vitro* method for determining the risk of occurrence of long-term kidney graft dysfunction in a subject under immunosuppressive treatment, comprising:
a) measuring the level of CD8⁺ T cells having a phenotype CD45RA⁺/CD197⁻ /CD27⁻/CD28⁻ in a blood sample of the subject,
b) comparing the level of CD8⁺ T cells having a phenotype CD45RA⁺/CD197⁻ /CD27⁻/CD28⁻ measured at step a) with one or more reference values of the level of CD8⁺ T cells having a phenotype CD45RA⁺/CD197⁻/CD27⁻/CD28⁻, and
c) determining the risk of occurrence of long-term kidney graft dysfunction in the said subject from the comparison performed at step b).

According to a main embodiment, this invention provides an *in vitro* method for determining the risk of occurrence of long-term kidney graft dysfunction in a subject currently presenting a stable graft function, comprising:
a) measuring the level of CD8⁺ T cells having a phenotype CD45RA⁺/CD197⁻ /CD27⁻/CD28⁻ in a blood sample of the subject,
b) comparing the level of CD8⁺ T cells having a phenotype CD45RA⁺/CD197⁻ /CD27⁻/CD28⁻ measured at step a) with one or more reference values of the level of CD8⁺ T cells having a phenotype CD45RA⁺/CD197⁻/CD27⁻/CD28⁻, and
c) determining the risk of occurrence of long-term kidney graft dysfunction in the said subject from the comparison performed at step b), wherein an increased level of CD8⁺ T cells having a phenotype CD45RA⁺/CD197⁻/CD27⁻/CD28⁻ is indicative of a risk of occurrence of long-term kidney graft dysfunction.

In another specific embodiment, this invention provides an *in vitro* method for determining the risk of occurrence of long-term kidney graft dysfunction in a subject under immunosuppressive treatment, currently presenting a stable graft function, comprising:
a) measuring the level of CD8⁺ T cells having a phenotype CD45RA⁺/CD197⁻ /CD27⁻/CD28⁻ in a blood sample of the subject,
b) comparing the level of CD8⁺ T cells having a phenotype CD45RA⁺/CD197⁻ /CD27⁻/CD28⁻ measured at step a) with one or more reference values of the level of CD8⁺ T cells having a phenotype CD45RA⁺/CD197⁻/CD27⁻/CD28⁻, and
c) determining the risk of occurrence of long-term kidney graft dysfunction in the said subject from the comparison performed at step b).

The term "immunosuppressive treatment" refers to the administration to the subject of immunosuppressive drugs or immunosuppressive agents or antirejection medications that inhibit or prevent activity of the immune system. They are used to prevent the rejection of transplanted organs. Biotherapies such as antibody-based therapies are often used.

As shown in Example 2, CD57⁺ CD8 T cells are preferentially found within the CD45RA⁺CD197⁻CD27⁻CD28⁻ T cells. CD57 expression correlates strongly with simultaneous expression with highly cytolytic/cytotoxic enzymes such as GZM-A (Granzyme A), GZM-B (Granzyme B), and PERF (Perforin).

Thus the invention provides an *in vitro* method for determining the risk of occurrence of long-term kidney graft dysfunction in a subject as defined above, which may further comprise measuring the level of CD57 in CD8+ T cells, in particular TEMRA CD8+ T cells, from the blood sample of the subject.

In view of the above, the invention further relates to an *in vitro* method, wherein:
- step a) comprises measuring the level of CD8⁺ T cells having a phenotype CD45RA⁺/CD197⁻/CD27⁻/CD28⁻/CD57⁺ in the blood sample of the subject, and
- step b) comprises comparing the level of CD8⁺ T cells having a phenotype CD45RA⁺/CD197⁻/CD27⁻/CD28⁻/CD57⁺ measured at step a) with one or more reference values of the level of CD8⁺ T cells having a phenotype CD45RA⁺/CD197⁻/CD27⁻/CD28⁻ /CD57⁺.

In the present specification, the name of each of the various markers of interest refers to the internationally recognized name of the corresponding gene, as found in internationally recognized gene sequences and protein sequences databases, including the database from the HUGO Gene Nomenclature Committee, that is available notably at the following Internet address http://www.genenames.org/.

In the present specification, the name of each of the markers of interest among CD8, CD45RA, CD197, CD27 and CD57 may also refer to the internationally recognized name of the corresponding gene, as found in the internationally recognized gene sequences and protein sequences database Genbank.

As used herein, CD8, which may also be termed "cluster of differentiation 8", is a co-receptor consisting of CD8 chains CD8A and/or CD8B, which is/are encoded respectively by the CD8A and CD8B genes.

As used herein, CD45RA, which may also be termed "cluster of differentiation 45RA" or "Protein tyrosine phosphatase, receptor type C" refers to the CD45RA isoform of the CD45 antigen and is encoded by the PTPRC gene.

As used herein, CD197, which may also be termed "cluster of differentiation 197" or "chemokine (C-C motif) receptor type 7" refers to the protein which is encoded by the CCR7 gene.

As used herein, CD27, which may also be termed "cluster of differentiation 27" or "tumor necrosis factor receptor superfamily, member 7" refers to the protein which is encoded by the CD27 gene.

As used herein, CD28, which may also be termed "cluster of differentiation 28" or "Tp44" refers to the protein which is encoded by the CD28 gene.

As used herein, CD57, which may also be termed "cluster of differentiation 57" or "beta-1,3-glucuronyltransferase 1 (glucuronosyltransferase P)" or "HNK-1" or "LEU7" refers to the protein which is encoded by the B3GAT1 gene.

As used herein, GZM-B, which may also be termed "Granzyme B" refers to the protein which is encoded by the GZMB gene.

As used herein, PRF1, which may also be termed "Perforin" or "Perforin-1", is a pore-forming protein which refers to the protein which is encoded by the PRF1 gene.

As used herein, TBX21, which may also be termed "T-bet" or "T-box transcription factor", or "T-box 21", refers to the protein which is encoded by the TBX21 gene.

According to the invention, "measuring the level of either one of the markers of the invention" comprises measuring the gene expression level of the marker at a nucleic acid and/or protein level, and preferably at a protein level.

Measuring the gene expression level may thus comprise or consist in (i) measuring the level of expression of a nucleic acid encoding for either one of the markers of the invention, and/or (ii) measuring the level of expression of a protein which is encoded by its corresponding gene.

According to a preferred embodiment, measuring the level of either one of the markers of the invention means measuring the expression of said markers at the surface of T cells. Alternatively, measuring the level of either one of the markers of the invention means measuring the expression of said markers within T cells.

Even more preferably, measuring the level of expression of a marker at the surface of T cells is achieved using flow cytometry, such as Fluorescence Activated Cell Sorting or FACS.

Alternatively, measuring the expression of a marker within T cells can also be achieved using flow cytometry, in particular FACS.

FACS method is well known in the Art and can be achieved as shown in the examples herein. Of course, the one skilled in the art will readily be able to adapt the method depending on the set of markers of which the level needs to be measured.

Step a) of the *in vitro* method for determining the risk of occurrence of long-term kidney graft dysfunction in a subject is preferably performed by measuring the combination of markers at the protein level. Most preferably, step a) of the *in vitro* method for determining the risk of occurrence of long-term kidney graft dysfunction in a subject is preferably performed by cytometry, including preferably cytofluorometry (FACS), since cytofluorometry allows measuring the simultaneous presence of the combination of protein markers on the same cell, and thus allows the most precise cell phenotype determination.

In particular, markers which are susceptible to be expressed at the surface of T cells include CD8, CD45RA, CD197, CD27, CD28 and CD57.

Markers which are susceptible to be expressed within T cells include Granzyme-B, Perforin, CD57 and T-bet.

Advantageously, an *in vitro* method for determining the risk of occurrence of long-term kidney graft dysfunction in a subject may further comprise a step of measuring the level of one or more additional markers selected in a group comprising Granzyme B, Perforin, CD57 and T-bet in CD8+ T cells, preferably TEMRA CD8+ T cells, from a blood sample of a subject.

According to a particular embodiment, the invention relates to an *in vitro* method for determining the risk of occurrence of long-term kidney graft dysfunction in a subject as defined above, which method further comprises measuring the level of Granzyme B in the CD8+ T cells from the blood sample of the subject.

According to a particular embodiment, the invention relates to an *in vitro* method for determining the risk of occurrence of long-term kidney graft dysfunction in a subject as defined above, which method further comprises a step of measuring the level of Perforin in the CD8+ T cells from the blood sample of the subject.

According to a particular embodiment, the invention relates to an *in vitro* method for determining the risk of occurrence of long-term kidney graft dysfunction in a subject as defined above, which method further comprises measuring the level of T-bet in the CD8+ T cells from the blood sample of the subject.

According to a particular embodiment, the invention relates to an *in vitro* method for determining the risk of occurrence of long-term kidney graft dysfunction in a subject as defined above, which method further comprises measuring the level of CD57 in the CD8+ T cells from the blood sample of the subject.

As shown in Examples 2 and 3, Granzyme-B and Perforin are highly cytotoxic enzymes, and CD57 expression correlates strongly with simultaneous expression of GZM-B (Granzyme B), and PERF (Perforin).

As shown in Example 4, T-bet relates to the T-box transcription factor and has been associated with effector phenotype and cytotoxic potential in resting CD8 T cells.

As shown in Example 4, the markers which have been defined above are comprised within a set of markers, linked phenotypically and functionally.

Thus, the invention also relates to an *in vitro* method for determining the risk of occurrence of long-term kidney graft dysfunction in a subject comprising a step of measuring the level of Granzyme B, Perforin, CD57 and/or T-bet in TEMRA CD57+ CD8+ T cells.

In some embodiments of the *in vitro* method above, step a) comprises measuring the level of CD8+ T cells having a phenotype selected in a group comprising:
- CD45RA⁺/CD197⁻/CD27⁻/CD28⁻ and CD57⁺,
- CD45RA⁺/CD197⁻/CD27⁻/CD28⁻ and Granzyme B⁺,
- CD45RA⁺/CD197⁻/CD27⁻/CD28⁻ and Perforin⁺,
- CD45RA⁺/CD197⁻/CD27⁻/CD28⁻ and T-bet⁺,
- CD45RA⁺/CD197⁻/CD27⁻/CD28⁻ and CD57⁺ and Granzyme B⁺,
- CD45RA⁺/CD197⁻/CD27⁻/CD28⁻ and CD57⁺ and Perforin⁺,
- CD45RA⁺/CD197⁻/CD27⁻/CD28⁻ and CD57⁺ and T-bet⁺,
- CD45RA⁺/CD197⁻/CD27⁻/CD28⁻ and CD57⁺ and Granzyme B⁺ and Perforin⁺,
- CD45RA⁺/CD197⁻/CD27⁻/CD28⁻ and CD57⁺ and Granzyme B⁺ and T-bet⁺,
- CD45RA⁺/CD197⁻/CD27⁻/CD28⁻ and CD57⁺ and Perforin⁺ and T-bet⁺,
- CD45RA⁺/CD197⁻/CD27⁻/CD28⁻ and CD57⁺ and Granzyme B⁺ and Perforin⁺ and T-bet⁺,
- CD45RA⁺/CD197⁻/CD27⁻/CD28⁻ and Granzyme B⁺, and Perforin⁺,
- CD45RA⁺/CD197⁻/CD27⁻/CD28⁻ and Granzyme B⁺, and T-bet⁺,
- CD45RA⁺/CD197⁻/CD27⁻/CD28⁻ and Perforin⁺ and T-bet⁺,
- CD45RA⁺/CD197⁻/CD27⁻/CD28⁻ and T-bet⁺, and Granzyme B⁺, and Perforin⁺.

In some embodiments of the *in vitro* method above, step a) comprises measuring the level of CD8+ T cells having a phenotype selected in a group comprising:
- CD3⁺/CD8⁺/CD45RA⁺/CD197⁻/CD27⁻/CD28⁻/CD57⁺/Tbet⁺,
- CD3⁺/CD8⁺/CD45RA⁺/CD197⁻/CD27⁻/CD28⁻/GZMb⁺/PERF⁺, and
- CD3⁺/CD8⁺/CD45RA⁺/CD197⁻/CD27⁻/CD28⁻/CD127⁻.

In some embodiments of the *in vitro* method above, step a) comprises measuring the level of CD8+ T cells having a phenotype selected in a group comprising:
- CD3⁺/CD8⁺/CD45RA⁺/CD197⁻/CD27⁻/CD28⁻.

The presence or absence of a given marker in the blood sample of a subject is summarized using a well-known terminology in the art. Thus when a given marker is detected, its corresponding name is followed by a "+" sign, such as in CD8+ or CD8⁺. Alternatively, when a given marker is not detected, its corresponding name is followed by a "-" sign, such as in CD8- or CD8⁻.

Of course, the threshold for which a given marker can be detected depends on the technique used for detection. Such a threshold may for instance be determined by the ability of a given ligand (i.e. an antibody) to fix the said marker.

In FACS, a population of cells expressing the said marker can be readily distinguished from a population of cells not expressing the said marker, within the same sample or with distinct samples for establishing a reference.

Alternatively, a given marker may be expressed at different levels. Such a case may for instance be observed for T-bet^{neg}, T-bet^{dull} and T-bet^{high} T cells (see Hersperger et al., Increased HIV-specific CD8+ T-cell cytotoxic potential in HIV elite controllers is associated with T-bet expression. Blood. 2011;117(14):3799-3808; see also Example 5).

Such a case may also be observed for PERF^{high}, PERF^{int}, or PERF^{low} T cells (see Example 4).

Accordingly, when such a case occurs, it is preferable to consider only a T cell population with the highest level of expression, or in that case T-bet^{high} and PERF^{high}, which are thus referred respectively as T-bet⁺ and PERF⁺.

This specification further describes an *in vitro* method for determining the risk of occurrence of long-term kidney graft dysfunction in a subject comprising:
a) measuring the level of CD8⁺ T cells having a phenotype CD45RA⁺/CD197⁻ /CD27⁻/CD28⁻/CD57⁺/T-bet⁺/GZM-B⁺/Perforin⁺ in a blood sample of the subject,
b) comparing the level of CD8⁺ T cells having a phenotype CD45RA⁺/CD197⁻ /CD27⁻/CD28⁻/CD57⁺/T-bet⁺/GZM-B⁺/Perforin⁺ measured at step a) with one or more reference values of the level of CD8⁺ T cells having a phenotype CD45RA⁺/CD197⁻/CD27⁻ /CD28⁻/CD57⁺/T-bet⁺/GZM-B⁺/Perforin⁺, and
c) determining the risk of occurrence of long-term kidney graft dysfunction in the said subject from the comparison performed at step b).

This invention further pertains to an *in vitro* method for determining the risk of occurrence of long-term kidney graft dysfunction in a subject, said subject being a kidney recipient with current stable graft function, comprising:
a) measuring the level of CD8⁺ T cells having a phenotype CD45RA⁺/CD197⁻ /CD27⁻/CD28⁻/CD57⁺/T-bet⁺/GZM-B⁺/Perforin⁺ in a blood sample of the subject,
b) comparing the level of CD8⁺ T cells having a phenotype CD45RA⁺/CD197⁻ /CD27⁻/CD28⁻/CD57⁺/T-bet⁺/GZM-B⁺/Perforin⁺ measured at step a) with one or more reference values of the level of CD8⁺ T cells having a phenotype CD45RA⁺/CD197⁻/CD27⁻ /CD28⁻/CD57⁺/T-bet⁺/GZM-B⁺/Perforin⁺, and
c) determining the risk of occurrence of long-term kidney graft dysfunction in the said subject from the comparison performed at step b).

The specification also describes a method for preventing long-term kidney graft dysfunction in a subject comprising:
a) performing the *in vitro* method as defined previously, in a blood sample of the subject, whereby the risk of occurrence of long-term kidney graft dysfunction in the subject is determined, and
b) administering to the subject a suitable therapeutic treatment.

According to another main embodiment, the invention relates to an immunosuppressive treatment for use in a method for preventing long-term kidney graft dysfunction in a subject; wherein the subject is a kidney recipient with current stable graft function, determined at risk for an occurence of long-term kidney graft dysfunction, as defined further above.

Of course either one of the *in vitro* methods for determining the risk of occurrence of long-term kidney graft dysfunction in a subject is suitable for the invention, and can be readily applied to a method for preventing long-term kidney graft dysfunction in a subject.

The "subject" is in particular a kidney recipient with current stable graft function, optionally under immunosuppressive treatment.

A suitable therapeutic treatment may be any method known in the Art to be useful for preventing long-term kidney graft dysfunction, such as an immunosuppressive treatment. Alternatively, the administration to the subject of a suitable therapeutic treatment may replace and/or adapt another pre-established treatment.

The adaptation of the immunosuppressive treatment may consist in: a modification of said immunosuppressive treatment if the subject has been determined as having a risk of occurrence of a renal dysfunction, which includes that the subject is "at risk" of developing a graft rejection, or a maintenance of said immunosuppressive treatment if the subject has been determined as having a low risk or no risk of occurrence of a kidney dysfunction, which includes that the subject has a low risk or has no risk of developing a graft rejection.

The present invention presents a major interest. It permits to diagnose or prognose (i.e. to identify), among patients under immunosuppressive treatment, those who are in the process of rejecting their graft and who could thus benefit from an adapted immunosuppressive treatment dedicated to stop or at least slow down the rejection process. Due to the non-reversible aspects of the damages caused by chronic rejection and the current late diagnosis of such phenotype, this achievement is really crucial and would allow a better management of the patients.

According to a particular embodiment, a method of the invention may further comprise a step of measuring the shape of the TCR Vbeta repertoire.

The shape of the TCR Vbeta repertoire at the genomic, transcriptomic or protein level is preferably determined independently for each Vβ family by any technology known in the art. For instance, the level of TCR transcripts of a particular Vβ family may be determined by calculating the ratio between these Vβ transcripts and the transcripts of a control housekeeping gene, such as the HPRT gene. The CDR3 length-distribution of a particular Vβ family may be determined by spectratyping, technology that determine frequency of each individual CDR3 length within a particular Vβ family). Preferably, in a graft rejecting subject, a significant percentage of Vβ families display an increase in their transcript numbers compared to patients with stable kidney graft function or to healthy individuals. in a graft rejecting subject, a significant percentage of Vβ families display an oligoclonal or monoclonal CDR3 length distribution compared to patients with stable kidney graft function or to healthy individuals. An example of methods to analyze T cell repertoire oligoclonality and/or the level of TCR transcripts, as well as scientific background relative to T cell repertoire, are clearly and extensively described in WO 02/084567 (24), which is herein incorporated by reference.

In particular, the level of TCR expression may be useful to determine restriction of the TCR Vβ repertoire diversity.

Advantageously, the detection of a restricted TCR Vβ repertoire within the method may be used as an additional control, as patients with restricted TCR Vβ repertoire have a higher amount of highly differentiated CD8 T cells.

The determination of the presence of a graft rejection or graft non-rejection phenotype is carried out thanks to the comparison of the obtained expression profile with a reference value from at least one reference expression profile in step (b).

A "reference expression profile" is a predetermined expression profile, obtained from a biological sample from a subject with a known particular graft state. In particular embodiments, the reference expression profile used for comparison with the test sample in step (b) may have been obtained from a biological sample from a group of healthy volunteers ("healthy reference expression profile") and/or from a biological sample from a graft rejecting subject ("rejecting reference expression profile"), and/or from a biological sample from a graft non-rejecting subject ("non-rejecting reference expression profile").

A reference expression profile may be determined either from one single subject, or from a panel of subjects with a known particular graft state or from a panel of healthy volunteers with a known well-functioning kidney.

For instance, a reference expression profile may be determined from one single reference subject, wherein the reference subject is different from the subject to be tested.

Alternatively, a reference expression profile may be determined from one single reference subject, wherein the reference subject is the same as the subject to be tested, but at a different timeline.

According to such embodiment, the *in vitro* method for determining the risk of occurrence of long-term kidney graft dysfunction in a subject may be used as a mean to follow the evolution of an expression profile over time.

Preferably, at least one reference expression profile is a rejecting reference expression profile (or long-term kidney graft rejection). Alternatively, at least one reference expression profile may be a non-rejecting reference expression profile. Alternatively, at least one reference expression profile may be a healthy reference expression profile. More preferably, the determination of the presence or absence of a graft rejection phenotype is carried out by comparison with at least one rejecting and at least one non-rejecting reference expression profiles or healthy reference expression profile. The diagnosis (or prognostic) may thus be performed using one rejecting reference expression profile and one non-rejecting reference expression profile or healthy reference expression profile. Advantageously, to get a stronger diagnosis, said diagnosis is carried out using several rejecting reference expression profiles and several non-rejecting reference expression profiles or several healthy reference expression profile.

The comparison of a tested subject expression profile with said reference expression profile(s) may be performed using statistical models or machine learning methods which aim is to predict a clinical response based on a combination of the explanatory variables (i.e. expression of markers of the invention) and demographic and clinical parameters (recipient age, donor age, recipient gender, donor gender, HLA (A⁺B⁺DR) mismatches (>4 vs. other), eGFR level at collection and PRA at inclusion).

In particular, a gene expression level may be measured at the genomic and/or nucleic and/or protein level, and preferably at a protein level.

Preferably, a "reference expression profile" is determined by measuring the level of expression of markers of the invention at the surface of T cells. Even more preferably, a "reference expression profile" is established using flow cytometry, such as FACS method.

The method according to the invention is dedicated for determining the risk of occurrence of kidney graft dysfunction in a subject within a "long term" period, which designates a follow up of the patients after their enrollment in the study of at least three years, preferably at least five years, more preferably at least seven years, eight years, nine years, ten years, eleven years, twelve years, thirteen years, fourteen years or at least fifteen years.

### Kits for determining the risk of occurrence of long-term kidney graft dysfunction

The specification describes a kit for determining the risk of occurrence of long-term kidney graft dysfunction in a subject, comprising one or more reagents for detecting or quantifying CD8, CD45RA, CD197, CD27 and/or CD28 level in T cells.

According to another main embodiment, the invention thus also relates to a use of a kit, as defined herein, comprising one or more reagents for detecting or quantifying CD45RA, CD197, CD27 and CD28 in CD8+ T cells; in an *in vitro* method for determining the risk of occurrence of long-term kidney graft dysfunction in a subject, said subject being a kidney recipient with current stable graft function.

The specification also describes a kit for determining the risk of occurrence of long-term kidney graft dysfunction in a subject, comprising one or more reagents for detecting or quantifying CD45RA, CD197, CD27 and/or CD28 level in CD8 T cells.

The specification also describes either one of the kits which are defined above further comprising one or more reagents for detecting or quantifying Granzyme B, Perforin and/or T-bet in CD8+ T cells.

The specification also describes either one of the kits which are defined above further comprising one or more reagents for detecting or quantifying Granzyme B in CD8+ T cells.

The specification also describes either one of the kits which are defined above further comprising one or more reagents for detecting or quantifying perforin in CD8+ T cells.

The specification also describes either one of the kits which are defined above further comprising one or more reagents for detecting or quantifying T-bet in CD8+ T cells.

The specification also describes either one of the kits which are defined above further comprising one or more reagents for detecting or quantifying CD57 in CD8+ T cells.

Such kit for determining the risk of occurrence of long-term kidney graft dysfunction in a subject may thus comprise at least one reagent for determination of the level of a marker on T cells, such as the markers of the invention, in particular CD8+ T cells, and more particularly TEMRA CD8+ T cells.

In a non-limitative manner, the kit may thus comprise ligands specific for CD3, CD8, CD45RA, CD197, CD27, CD28, CD57, CD127, T-bet, Perforin and/or Granzyme-B.

In particular, a ligand may be an antibody.

References for said antibodies may vary depending on the method used for determining the level of said markers.

Accordingly, when the method for determining the level of said markers is flow cytometry, one may for instance refer to the antibodies which have been described in the examples.

Of course, combinations of said ligands are also considered by the invention, as well as modified ligands. As an example, antibodies which linked covalently or non-covalently to a detectable molecule are also considered such as CD3-VioBlue, CD8-VioGreen, CD45RA-APC-Vio770, CD127-PE and CD57-FITC antibodies which have been described as well in the examples.

In addition, other reagents may be included in said kits, such as reagents for staining dead cells, and/or reagents or any other sample, or biological sample, which may be used for the purposes of an internal control, such as a reference blood sample.

Such kit for determining the risk of occurrence of long-term kidney graft dysfunction in a subject may also further comprise at least one reagent for determination of at least one additional parameter useful for the detection, such as standard biological parameters specific for said subject grafted organ type (notably the presence of anti-HLA antibodies), phenotypic analyses of peripheral blood cells, and quantitative and/or qualitative analysis of peripheral blood cells immune repertoire (such as the T cell repertoire oligoclonality and the level of TCR transcripts).

In some embodiments, such kit further comprises a support containing the values of a "reference expression profile" for the purpose of using the kit for performing an *in vitro* method for determining the risk of occurrence of long-term kidney graft dysfunction in a subject that is described in the present specification. The support containing the values of a "reference expression profile" may be selected in a group of supports comprising an analog technical information support such a printed solid support, or a digital information support such as a digital information storage device, which includes a digital information storage memory, a CD, a DVD, etc.

### EXAMPLES

### A. MATERIAL & METHODS

### Subjects and Ethics statement

The University Hospital Ethical Committee and the Committee for the Protection of Patients from Biological Risks approved the study. All age-matched kidney transplant patients gave informed consent. 131 transplant recipients who had received a first and unique kidney transplant from deceased donor and displayed a stable graft function (MDRD >40mL/min, proteinuria <1g/24h) for at least 5 years were enrolled. Patients were prescreened and designed as stable according to an estimated glomerular filtration rate (eGFR) above 40 ml/min, a stable creatinemia (±25% of the mean value of creatinemia in the year before the inclusion) and a daily proteinuria <1 g/day. These criteria had to be confirmed at 3 months after the enrollment of the patients to confirm the stability of the patient. Patients received tacrolimus or cyclosporine A (CNI) for maintenance therapy with or without mycophenolatemofetil (MMF), azathioprine (AZA) and/or steroids. All patients were compliant with the medical description and have not undergone an episode of rejection or an ongoing infection during the monitoring period. At the inclusion, anti-class I PRA was detected for 3 patients and anti-class II PRA for 6 patients, including 2 patients exhibited both. Graft dysfunction was assessed independently by two nephrologists and defined by at least 3 values of creatinemia above 25% of the basal level within the last 2 years of follow-up and/or a proteinuria >1g/24h. Two groups of patients were studied: Kidney recipients under standard immunosuppression with stable graft function and (i) without TCR Vβ alteration (diverse-STABLE; n=86) or (ii) with TCR Vβ alteration (restricted-STABLE; n=45).

### Study Design

131 patients were prospectively recruited according to the exclusion/inclusion criteria described in the Subjects and Ethics statement section (See above and Figure 1). At the time of the inclusion, TREC level was measured for all patients as well as TCR Vβ repertoire using TcLandscape®. An unsupervised statistical method (i-e a method aim to identify subgroup of patients based on expression of markers without prior knowledge of the existence of different groups of patients) (Miqueu et al. Analysis of the peripheral T-cell repertoire in kidney transplant patients. Eur. J. Immunol. 2010;40(11):3280-3290) was used to stratify patients into two arms according to the alteration of the TCR Vβ repertoire (diverse-STABLE, n=86; restricted-STABLE, n=45). Among the 131 patients recruited, frozen PBMC were available for 106 patients (diverse-STABLE, n=71; restricted-STABLE, n=35) and were subjected to detailed phenotypic and functional analyses. Within each arm, a fraction of the patients were selected to characterize the transcripts expression of a selected CD8-related genes set (diverse-STABLE, n=17; restricted-STABLE, n=12). The selection of the patients was done in order to adjust the demographic and clinical parameters (time post-transplantation, recipient age and gender, donor age and gender, and HLA mismatches).

### Blood samples

Peripheral blood mononuclear cells (PBMC) were separated on a Ficoll gradient layer and frozen in DMSO-10% autologous serum.

### Polychromatic flow cytometry/FACS

Cells were analyzed with a LSRII flow cytometer (BD Immunocytometry Systems). 2×10⁶ frozen PBMCs were surface stained with antibodies specific for CD3 (BW264/56; VioBlue), CD8 (BW135/80; VioGreen), CD45RA (T6D11; APC-Vio770), CD197 (3D12; PE-Cy7), CD27 (L128; Brilliant Violet 605) and CD28 (CD28.2; PE-CF594). In addition to this core-staining cocktail, different combinations of antibody were used CD127 (MB15-18C9; PE), CD57 (TB03; FITC), T-bet (04-46; PE), Granzyme B (GB11; Alexa Fluor 700), and Perforin (B-D48; PE). Yellow LIVE/DEAD Fixable Dead Cell Stain Kit was used to exclude dead cells from analysis. BD CompBeads stained separately with individual mAbs were used to define the compensation matrix. Data were analyzed using FlowJo Version 9.0.1 (TreeStar). All the antibodies were purchased from BD Biosciences except for CD3-VioBlue, CD8-VioGreen, CD45RA-APC-Vio770, CD127-PE, and CD57-FITC (Miltenyi) and Perforin-PE (Diaclone).

### Functional assays

PBMC were thawed and rested overnight in complete RPMI medium (10% FSC). PBMC were then washed and the cell concentration was adjusted at 2x10⁶ cells/mL in complete RPMI. Cells were stimulated for 6h with coated anti-CD3 (3µg/mL) and anti-CD28.2 mAb (5µg/mL) in a final volume of 1mL in 24 wells flat-bottom plate. When indicated PE-conjugated anti-CD107a mAb (10µL/well) was added at the beginning of the culture. After 2h, monensin (1µg/mL; in well with anti-CD107a mAb) or brefeldin A (5ug/mL) were added. PBMCs were stained for cell surface markers (CD3, CD8, CD45RA, CD197, CD27 and CD28), fixed and permeabilized according to the manufacture procedure (eBiosciences) and then stained for IFNγ (Alexa Fluor 700), TNFα (FITC) and T-bet (O4-46; PE). A minimal of 1x10⁴ CD3⁺CD8⁺ cells was recorded (median 8x10⁵ - range 1x10⁵ - 2x10⁷).

### Statistical methods

***Descriptive analysis.*** Quantitative variables were compared using a t-test and the p-value was corrected according to the Holm's procedure. Qualitative variables were compared using a χ² test. Patients with 4 or more HLA-A, -B, -DR mismatches were considered as highly incompatible patients (*p-values<0.05).

***Survival analysis.*** The principal endpoint was the time between the fifth anniversary of transplantation and the time of graft dysfunction. A non-parametric estimator of survival function for truncated and censored data was used (Pan et al., A non-parametric estimator of survival functions for arbitrarily truncated and censored data. Lifetime Data Anal. 1998;4(2): 187-202). A Cox model adapted to truncated data was used for multivariate analysis (Therneau et al. Modeling Survival Data: Extending the Cox Model (Statistics for Biology and Health). Springer; 2010). The Wald's test was used to evaluate the significance of hazard ratios.

***sjTREC analysis.*** Multivariate analysis was used to evaluate the confounding factors using a generalized linear model with a gamma distribution and inversed link function (see McCullagh et al. Generalized Linear Models, Second Edition (Chapman & Hall/CRC Monographs on Statistics & Applied Probability) 2nd Edition; 1989).

***CD8 T cell phenotype and function analysis.*** Analysis and presentation of distributions of the CD8 T cells phenotype were performed using Simplified Presentation of Incredibly Complex Evaluations (SPICE version 5.1, downloaded from http://exon.niaid.nih.gov) (see also Roederer et al. SPICE: exploration and analysis of post-cytometric complex multivariate datasets. Cytometry. 2011;79A(2):167-174). Holm multiple comparison test was used to compare the expression of phenotypic and functional markers (Holm et al. Simple Sequentially Rejective Multiple Test Procedure. Scandinavian Journal of Statistics. 1979;6(2):65-70).

### B. RESULTS

### Example 1. Reduction in TCR Vβ repertoire diversity is associated with an increase of highly differentiated TEMRA (CD45RA⁺CD197⁻CD27⁻CD28⁻) CD8 effector T cells.

Among the 131 patients with a stable graft function under standard biotherapy immunosuppression at the time of inclusion (median time post-transplantation 7.78 years - range 5.01-21.66), 45 exhibited a restricted TCR Vβ repertoire at inclusion in the study (median time post-transplantation 6.55 years - range 5.11-19.58) and 86 did not (median time post-transplantation 8.10 years - range 5.01-21.66) (see Figure 1). The stratification of restricted vs. diverse TCR Vβ repertoire was based on the results obtained using a previously published unsupervised statistical method (i-e a method aim to identify subgroup of patients based on expression of markers without prior knowledge of the existence of different groups of patients) (positive value of the first Principal Component Analysis Covariate 1; PCA1). By measuring a broad array of phenotypic and functional proteins, we sought a deeper understanding of CD8 T cells among transplanted patients who display differential TCR Vβ repertoire diversity. CD8 T cells were described using a panel of multiple staining that was designed to simultaneously cover the phenotype and the function of CD8 T cells.

CD8 T cells were first classified as **naive** (CD45RA⁺CD197⁺), **central memory** (CD45RA⁻CD197⁺; CM), **effector memory** (CD45RA⁻CD197⁻; EM) or **terminally differentiated effector memory** (CD45RA⁺CD197⁻; TEMRA).

Representative gating schemes are shown in **Figure 2****.**

### Results

Patients with a restricted TCR Vβ repertoire exhibit a higher frequency of TEMRA CD8 T cells as compared to patients with a diverse TCR Vβ repertoire (52.74±2.96 vs. 31.39±1.99 respectively; p<0.001; Figure 3A) and a decrease in the frequency of naive CD8 T cells (14.13±1.62 vs. 29.31±1.82 respectively; p<0.001; Figure 3A).

Based on CD28 and CD27 expression, three differentiated phenotypes can be identified: early (CD27⁺CD28⁺), **intermediate** (CD28⁻CD27⁺) and **late** (CD28⁻CD27⁻). Such classification is known in the Art (see for reference: Appay et al. Memory CD8+ T-cells vary in differentiation phenotype in different persistent virus infections. Nat. Med. 2002;8(4):379-385).

A restricted TCR Vβ repertoire was associated with a marked increase in CD27⁻CD28⁻ CD8 T cells (55.13±3.14 vs. 23.06±2.30 respectively; p<0.001; Figure 3B). Finally, when the expression of CD27 and CD28 was analyzed within the 4 subsets previously identified (**CM, EM, Naive** and **TEMRA**), an increase in CD27⁻CD28⁻ T cells was associated with a decrease in CD27⁺CD28⁺ T cells in EM and TEMRA CD8 T cells in restricted TCR Vβ repertoire patients (p<0.001; Figure 3C).

### Conclusion

### Collectively, the restriction in TCR Vβ diversity is associated with an expansion of TEMRA cells with highly differentiated phenotype.

### Example 2. CD8 T cells in patients with restricted TCR Vβ repertoire demonstrated a high expression of cytotoxic molecules (granzyme B and perforin) compared with patients with a polyclonal TCR Vβ repertoire.

Late differentiated phenotype is associated with a high cytotoxic capacity, characterized notably by the expression of cytotoxic molecules such as granzyme B and perforin (see Appay et al. Memory CD8+ T cells vary in differentiation phenotype in different persistent virus infections. Nat. Med. 2002;8(4):379-385; Hamann et al. Phenotypic and functional separation of memory and effector human CD8+ T cells. J. Exp. Med. 1997;186(9):1407-1418).

### Results

On average, 64% of CD8 T cells in patients with polyclonal TCR Vβ repertoire express none of the cytolytic molecules whereas the frequency of GZM-B⁻PERF⁻ was only of 39% in patients with a restricted TCR Vβ repertoire (p=1.67x10⁻⁷; Figure 4). A significant increase of CD8 T cells expressing either GZM-B only (28.04±3.05; p=0.007) or GZM-B and PERF 30.61±2.83; p=5.82x10⁻⁶) was observed in patients with a restricted TCR Vβ repertoire. Three levels of expression of PERF could be observed within CD8 T cells (PERF^{neg}, PERF^{int} and PERF^{high}; Figure 4B). PERF^{neg} CD8 T cells were preferentially found within the naive CD45RA⁺CD197⁺ and EM CD45RA⁻CD197⁻ subsets, and co-expressed CD27 and CD28 cells (Figure 4B). A gradual enrichment of differentiated CD8 T cells (CD27⁻CD28⁻) combined with high frequency of TEMRA CD45RA⁺CD197⁻ was observed with the increase level of PERF expression (Figure 4B). CD8 T cells with a restricted TCR Vβ repertoire exhibit a higher expression of PERF as compared to patients with a diverse TCR Vβ repertoire (PERF^{hi} 21.04±2.80 vs. 7.84±0.88 respectively; p=0.0001; Figure 4C). The enhanced expression of PERF was associated with an increase in the Mean Fluorescence Intensity (MFI) of GZM-B (diverse TCR Vβ repertoire 4375±487 vs. restricted TCR Vβ repertoire 5809±283; p=0.0078; Figure 4D). Finally, a positive correlation between the expression of PERF and GZM-b was observed (r²=0.4622, p<0.0001).

High cytolytic potential can be readily measured using the expression of CD57, as described in the literature (see Chattopadhyay et al. The cytolytic enzymes granyzme A, granzyme B, and perforin: expression patterns, cell distribution, and their relationship to cell maturity and bright CD57 expression. Journal of Leukocyte Biology. 2008;85(1):88-97; Brenchley et al. Expression of CD57 defines replicative senescence and antigen-induced apoptotic death of CD8+ T cells. Blood. 2002;101(7):2711-2720) as CD57 expression correlates strongly with simultaneous expression of GZM-A (Granzyme A), GZM-B (Granzyme B), and PERF (Perforin)..

Patients with restricted TCR Vβ repertoire display a higher frequency of CD57⁺ CD8 T cells as compared to patients with a diverse TCR Vβ repertoire (47.75±2.69 vs. 26.83±1.59 % respectively; p=9.13x10⁻⁹; Figure 4E). CD57⁺ CD8 T cells were preferentially found within the CD45RA⁺CD197⁻CD27⁻CD28⁻ T cells *(**Figure 8**).*

Finally, the cytolytic activity of CD8 T cells was assessed after short-term polyclonal stimulation (6h with plate bound aCD3 aCD28.2 mAb) of PBMC purified from patients with a restricted or a diverse TCR Vβ repertoire. Patients with a restricted TCR Vβ repertoire rapidly released cytotoxic granules as assessed by the upregulation of CD107a at the cell surface of CD8 T cells (7.11±2.35 vs. 1.69±0.65 % respectively; p=0.0033; Figure 4F).

### Conclusion

Collectively, the restriction of TCR Vβ repertoire is associated with an accumulation of TEMRA CD8 T cells with high cytolytic potential (CD57⁺GZM-b⁺PERF⁺). This result provides evidence that the *in vitro* method of the invention may advantageously comprise a step of measuring the level of CD57, Granzyme B and/or perforin in on CD8+ T cells.

### Example 3. CD8 T cells in patients with restricted TCR Vβ repertoire expressed higher levels of T-bet than patients with diverse TCR Vβ repertoire.

Expression of the T-box transcription factor T-bet has been associated with effector phenotype and cytotoxic potential in resting CD8 T cells. As previously reported, three populations can be defined based on the expression of T-bet: T-bet^{neg}, T-bet^{dull} and T-bet^{high} (see Hersperger et al., Increased HIV-specific CD8+ T-cell cytotoxic potential in HIV elite controllers is associated with T-bet expression. Blood. 2011;117(14):3799-3808).

Whereas the frequency of T-bet^{dull} CD8 T cells was similar between patients with diverse or restricted TCR Vβ repertoire, patients with a restricted TCR Vβ repertoire exhibit a marked increase in T-bet^{high} CD8 T cells (44.05±4.05 vs. 25.25±1.88 % respectively; p=0.0002; Figure 5A).

We took advantage of the co-staining with CD45RA, CD197, CD27, CD28 and CD57 to better characterize the T-bet^{neg} and T-bet^{high} in the whole cohort of kidney transplant recipients.

### Results

Expression of T-bet^{high} was positively correlated with the expression of CD57 (R²=0.4012; p<0.0001; Figure 5B). Most of the T-bet^{high} CD8 T cells which express the effector-associated marker CD57 lose the expression of CD27 and CD28 and are preferentially found within EM and TEMRA CD8 subset (Figure 5C).

Next, we compared the phenotype of T-bet^{high} CD8 T cells according to the usage of the TCR Vβ repertoire (Figure 5D). Patients with a restricted TCR Vβ repertoire exhibit T-bet^{high} CD8 T cells with an increased expression of CD57 (67.37±2.34 vs. 52.86±2.13% respectively; p=1.47x10⁻⁵; Figure 5D), associated with a highly differentiated phenotype CD27⁻CD28⁻ T cells (75.57±2.50 vs. 44.76±3.26 % respectively; p=9.26x10⁻¹¹; Figure 5D) and an increase in TEMRA CD45RA⁺CD197⁻ T cells (59.45±3.06 vs. 48.00±2.67 % respectively; p=0.018; Figure 5D).

### Conclusion

This result provides evidence that the *in vitro* method of the invention may advantageously comprise a step of measuring the level of T-bet in TEMRA CD8+ T cells.

### Example 4: Increase of highly differentiated TEMRA CD8 T cells is a risk factor for graft dysfunction

In view of the broad number of parameters measured on CD8 T cells, we determined a link between the accumulation of highly differentiated CD8 T cells and graft-function outcome.

### Results

From an homogenous population of patients with a stable graft function under immunosuppressive treatment, we first identified two groups of patients based on an unsupervised hierarchical cluster analysis (i-e a method aim to identify subgroup of patients based on expression of markers without prior knowledge of the existence of different groups of patients) of the different markers measured on CD8 T cells.

A set of markers, linked phenotypically and functionally, (TEMRA⁺ CD27⁻ CD28⁻ CD57⁺T-bet⁺ GZM-b⁺Perforin⁺) allows for the segregation of the population into two groups (54 patients with an increase of differentiated TEMRA CD8 T cells, (or TCD8 "left") and 50 patients without an increase; TCD8 "right"; Figure 6A). With more than 7 years of follow-up since inclusion time, we then assessed whether the kidney dysfunction was associated with the stratification of patients based on markers expressed by CD8 T cells.

The probability of late graft dysfunction during the 10 years post-inclusion was of 41.6% (CI95%= 20.7%-57.0%) for patients with an increase of highly differentiated TEMRA CD8 T cells whereas this risk was only of 23.0% (CI95%= 6.5%-36.5%) for the other group of patients (see Figure 6B).

A limited set of factors was included in the multivariate regression model (time post-transplantation, recipient and donor age, recipient and donor gender, number of HLA mismatches above 4; panel-reactive antibody (or PRA) at inclusion and estimated glomerular filtration rate (eGFR) at inclusion). Well-known risk-factors (episodes of acute or antibody mediated rejection) were defined as exclusion criteria and thus were not used in the model. Using multivariate regression, we showed that patients with an increase of highly differentiated TEMRA CD8 T cells had a 1.96 fold higher risk of graft dysfunction during their follow-up and before their 15^{th} graft anniversary (p=0.0621).

The values of PCA1 (or Principal Component Analysis Covariate 1) that reflects the shape of the TCR Vβ repertoire (PCA1>0: restricted TCR Vβ repertoire; PCA1<0: diverse TCR Vβ repertoire) confirm this result as patients with a restricted TCR Vβ repertoire had a 1.52 fold higher risk of graft dysfunction during their follow-up and before their 15^{th} graft anniversary (p=0.3015).

### Conclusion

Collectively, the monitoring of differentiated TEMRA CD8+ T cells allows the early identification of patients with higher risk of graft dysfunction, from an homogenous population of patients currently under immunosuppressive treatment, with a stable graft function.

## Claims

1. An *in vitro* method for determining the risk of occurrence of long-term kidney graft dysfunction in a subject, said subject being a kidney recipient with current stable graft function, comprising:
a) measuring the level of CD8⁺ T cells having a phenotype CD45RA⁺/CD197⁻ /CD27⁻/CD28⁻ in a blood sample of the subject,
b) comparing the level of CD8⁺ T cells having a phenotype CD45RA⁺/CD197⁻ /CD27⁻/CD28⁻ measured at step a) with one or more reference values of the level of CD8⁺ T cells having a phenotype CD45RA⁺/CD197⁻/CD27⁻/CD28⁻, and
c) determining the risk of occurrence of long-term kidney graft dysfunction in the said subject from the comparison performed at step b), wherein an increased level of CD8⁺ T cells having a phenotype CD45RA⁺/CD197⁻/CD27⁻/CD28⁻ is indicative of a risk of occurrence of long-term kidney graft dysfunction.

2. The *in vitro* method according to claim 1, wherein:
- step a) comprises measuring the level of CD8⁺ T cells having a phenotype CD45RA⁺/CD197⁻/CD27⁻/CD28⁻/CD57⁺ in the blood sample of the subject, and
- step b) comprises comparing the level of CD8⁺ T cells having a phenotype CD45RA⁺/CD197⁻/CD27⁻/CD28⁻/CD57⁺ measured at step a) with one or more reference values of the level of CD8⁺ T cells having a phenotype CD45RA⁺/CD197⁻/CD27⁻/CD28⁻ /CD57⁺.

3. The *in vitro* method according to any one of claims 1 and 2, further comprising measuring the level of Granzyme B in the CD8+ T cells from the blood sample of the subject.

4. The *in vitro* method according to any one of claims 1 to 3, further comprising measuring the level of Perforin in the CD8+ T cells from the blood sample of the subject.

5. The *in vitro* method according to any one of claims 1 to 4, further comprising measuring the level of T-bet in the CD8+ T cells from the blood sample of the subject.

6. The *in vitro* method according to claim 1, wherein step a) comprises measuring the level of CD8+ T cells having a phenotype selected in a group comprising:
- CD45RA⁺/CD197⁻/CD27⁻/CD28⁻ and CD57⁺,
- CD45RA⁺/CD197⁻/CD27⁻/CD28⁻ and Granzyme B⁺,
- CD45RA⁺/CD197⁻/CD27⁻/CD28⁻ and Perforin⁺,
- CD45RA⁺/CD197⁻/CD27⁻/CD28⁻ and T-bet⁺,
- CD45RA⁺/CD197⁻/CD27⁻/CD28⁻ and CD57⁺ and Granzyme B⁺,
- CD45RA⁺/CD197⁻/CD27⁻/CD28⁻ and CD57⁺ and Perforin⁺,
- CD45RA⁺/CD197⁻/CD27⁻/CD28⁻ and CD57⁺ and T-bet⁺,
- CD45RA⁺/CD197⁻/CD27⁻/CD28⁻ and CD57⁺ and Granzyme B⁺ and Perforin⁺,
- CD45RA⁺/CD197⁻/CD27⁻/CD28⁻ and CD57⁺ and Granzyme B⁺ and T-bet⁺,
- CD45RA⁺/CD197⁻/CD27⁻/CD28⁻ and CD57⁺ and Perforin⁺ and T-bet⁺,
- CD45RA⁺/CD197⁻/CD27⁻/CD28⁻ and CD57⁺ and Granzyme B⁺ and Perforin⁺ and T-bet⁺,
- CD45RA⁺/CD197⁻/CD27⁻/CD28⁻ and Granzyme B⁺, and Perforin⁺,
- CD45RA⁺/CD197⁻/CD27⁻/CD28⁻ and Granzyme B⁺, and T-bet⁺,
- CD45RA⁺/CD197⁻/CD27⁻/CD28⁻ and Perforin⁺ and T-bet⁺,
- CD45RA⁺/CD197⁻/CD27⁻/CD28⁻ and T-bet⁺, and Granzyme B⁺, and Perforin⁺.

7. The *in vitro* method according to claim 1, wherein the subject is a kidney recipient with current stable graft function and under immunosuppressive treatment.

8. An immunosuppressive treatment for use in a method for preventing long-term kidney graft dysfunction in a subject; wherein the subject is a kidney recipient with current stable graft function, determined at risk for an occurence of long-term kidney graft dysfunction according to any one of claims 1 to 7.

9. Use of a kit comprising one or more reagents for detecting or quantifying CD45RA, CD197, CD27 and CD28 in CD8+ T cells; in an *in vitro* method for determining the risk of occurrence of long-term kidney graft dysfunction in a subject, said subject being a kidney recipient with current stable graft function.

## Patentansprüche

1. *In-vitro*-Verfahren zur Bestimmung des Risikos des Auftretens einer langfristigen Nierentransplantat-Dysfunktion bei einem Individuum, wobei das Individuum ein Nierenempfänger mit aktuell stabiler Transplantatfunktion ist, umfassend:
a) das Messen der Menge an CD8⁺-T-Zellen mit einem Phänotyp CD45RA⁺/CD197⁻/CD27⁻/CD28⁻ in einer Blutprobe des Individuums,
b) das Vergleichen der in Schritt a) gemessenen Menge an CD8⁺-T-Zellen mit einem Phänotyp CD45RA⁺/CD197⁻ /CD27⁻/CD28⁻ mit einem oder mehreren Referenzwerten der Menge an CD8⁺-T-Zellen mit einem Phänotyp CD45RA⁺/CD197⁻ /CD27⁻/CD28⁻, und
c) das Bestimmen des Risikos des Auftretens einer langfristigen Nierentransplantat-Dysfunktion in dem Individuum aus dem in Schritt b) durchgeführten Vergleich, wobei eine erhöhte Menge an CD8⁺-T-Zellen mit einem Phänotyp CD45RA⁺/CD197⁻/CD27⁻/CD28⁻ auf ein Risiko des Auftretens einer langfristigen Nierentransplantat-Dysfunktion hinweist.

2. *In* vitro-Verfahren nach Anspruch 1, wobei:
- Schritt a) das Messen der Menge an CD8⁺-T-Zellen mit einem Phänotyp CD45RA⁺/CD197⁻/CD27⁻/CD28⁻/CD57⁺ in der Blutprobe des Individuums umfasst, und
- Schritt b) das Vergleichen der in Schritt a) gemessenen Menge an CD8⁺-T-Zellen mit einem Phänotyp CD45RA⁺/CD197⁻ /CD27⁻/CD28⁻/CD57⁺ mit einem oder mehreren Referenzwerten der Menge an CD8⁺-T-Zellen mit einem Phänotyp CD45RA⁺/CD197⁻/CD27⁻/CD28⁻/CD57⁺ umfasst.

3. In-vitro-Verfahren nach einem der Ansprüche 1 und 2, zudem umfassend das Messen der Menge an Granzym B in den CD8⁺-T-Zellen aus der Blutprobe des Individuums.

4. In-vitro-Verfahren nach einem der Ansprüche 1 bis 3, zudem umfassend das Messen der Menge an Perforin in den CD8⁺-T-Zellen aus der Blutprobe des Individuums.

5. *In* vitro-Verfahren nach einem der Ansprüche 1 bis 4, zudem umfassend das Messen der Menge an T-bet in den CD8⁺-T-Zellen aus der Blutprobe des Individuums.

6. *In* vitro-Verfahren nach Anspruch 1, wobei Schritt a) das Messen der Menge an CD8⁺-T-Zellen mit einem Phänotyp umfasst, der ausgewählt ist aus einer Gruppe, umfassend:
- CD45RA⁺/CD197⁻/CD27⁻/CD28⁻ und CD57⁺,
- CD45RA⁺/CD197⁻/CD27⁻/CD28⁻ und Granzym B⁺,
- CD45RA⁺/CD197⁻/CD27⁻/CD28⁻ und Perforin⁺,
- CD45RA⁺/CD197⁻/CD27⁻/CD28⁻ und T-bet⁺,
- CD45RA⁺/CD197⁻/CD27⁻/CD28⁻ und CD57⁺ und Granzym B⁺,
- CD45RA⁺/CD197⁻/CD27⁻/CD28⁻ und CD57⁺ und Perforin⁺,
- CD45RA⁺/CD197⁻/CD27⁻/CD28⁻ und CD57⁺ und T-bet⁺,
- CD45RA⁺/CD197⁻/CD27⁻/CD28⁻ und CD57⁺ und Granzym B⁺ und Perforin⁺ ,
- CD45RA⁺/CD197⁻/CD27⁻/CD28⁻ und CD57⁺ und Granzym B⁺ und T-bet⁺,
- CD45RA⁺/CD197⁻/CD27⁻/CD28⁻ und CD57⁺ und Perforin⁺ und T-bet⁺,
- CD45RA⁺/CD197⁻/CD27⁻/CD28⁻ und CD57⁺ und Granzym B⁺ und Perforin⁺ und T-bet⁺,
- CD45RA⁺/CD197⁻/CD27⁻/CD28⁻ und Granzym B⁺ und Perforin⁺,
- CD45RA⁺/CD197⁻/CD27⁻/CD28⁻ und Granzym B⁺ und T-bet⁺,
- CD45RA⁺/CD197⁻/CD27⁻/CD28⁻ und Perforin⁺, und T-bet⁺,
- CD45RA⁺/CD197⁻/CD27⁻/CD28⁻ und T-bet⁺ und Granzym B⁺ und Perforin⁺ .

7. In-vitro-Verfahren nach Anspruch 1, wobei das Individuum ein Nierenempfänger mit aktuell stabiler Transplantatfunktion und unter immunsuppressiver Behandlung ist.

8. Immunsuppressive Behandlung zur Verwendung bei einem Verfahren zur Verhinderung einer langfristigen Nierentransplantat-Dysfunktion bei einem Individuum, wobei das Individuum ein Nierenempfänger mit aktuell stabiler Transplantatfunktion ist, bei dem ein Risiko für das Auftreten einer langfristigen Nierentransplantat-Dysfunktion nach einem der Ansprüche 1 bis 7 festgestellt wurde.

9. Verwendung eines Kits, das ein oder mehrere Reagenzien zum Nachweis oder zur Quantifizierung von CD45RA, CD197, CD27 und CD28 in CD8⁺-T-Zellen umfasst; bei einem In-vitro-Verfahren zur Bestimmung des Risikos für das Auftreten einer langfristigen Nierentransplantat-Dysfunktion bei einem Individuum, wobei das Individuum ein Nierenempfänger mit aktuell stabiler Transplantatfunktion ist.

## Revendications

1. Procédé *in vitro* pour la détermination du risque d'occurrence de dysfonctionnement de greffon rénal à long terme chez un sujet, ledit sujet étant un receveur de rein à fonction de greffon pour le moment stable, comprenant :
a) la mesure du taux de lymphocytes T CD8⁺ ayant un phénotype CD45RA⁺/CD197⁻/CD27⁻/CD28⁻ dans un échantillon de sang du sujet,
b) la comparaison du taux de lymphocytes T CD8⁺ ayant un phénotype CD45RA⁺/CD197⁻/CD27⁻/CD28⁻ mesuré à l'étape a) avec une ou plusieurs valeurs de référence du taux de lymphocytes T CD8⁺ ayant un phénotype CD45RA⁺/CD197⁻/CD27⁻/CD28⁻ et
c) la détermination du risque d'occurrence de dysfonctionnement de greffon rénal à long terme chez ledit sujet à partir de la comparaison effectuée à l'étape b),
dans lequel un taux accru de lymphocytes T CD8⁺ ayant un phénotype CD45RA⁺/CD197⁻/CD27⁻/CD28⁻ est révélateur d'un risque d'occurrence de dysfonctionnement de greffon rénal à long terme.

2. Procédé *in vitro* selon la revendication 1, dans lequel :
- l'étape a) comprend la mesure du taux de lymphocytes T CD8⁺ ayant un phénotype CD45RA⁺/CD197⁻/CD27⁻/CD28⁻/CD57⁺ dans l'échantillon de sang du sujet et
- l'étape b) comprend la comparaison du taux de lymphocytes T CD8⁺ ayant un phénotype CD45RA⁺/CD197⁻/CD27⁻/CD28⁻/CD57⁺ mesuré à l'étape a) avec une ou plusieurs valeurs de référence du taux de lymphocytes T CD8⁺ ayant un phénotype CD45RA⁺/CD197⁻/CD27⁻/CD28⁻/CD57⁺.

3. Procédé *in vitro* selon l'une quelconque des revendications 1 et 2, comprenant en outre la mesure du taux de granzyme B dans les lymphocytes T CD8⁺ provenant de l'échantillon de sang du sujet.

4. Procédé *in vitro* selon l'une quelconque des revendications 1 à 3, comprenant en outre la mesure du taux de perforine dans les lymphocytes T CD8⁺ provenant de l'échantillon de sang du sujet.

5. Procédé *in vitro* selon l'une quelconque des revendications 1 à 4, comprenant en outre la mesure du taux de T-bet dans les lymphocytes T CD8⁺ provenant de l'échantillon de sang du sujet.

6. Procédé *in vitro* selon la revendication 1, dans lequel l'étape a) comprend la mesure du taux de lymphocytes T CD8⁺ ayant un phénotype choisi dans un groupe comprenant :
- CD45RA⁺/CD197⁻/CD27⁻/CD28⁻ et CD57⁺
- CD45RA⁺/CD197⁻/CD27⁻/CD28⁻ et granzyme B⁺,
- CD45RA⁺/CD197⁻/CD27⁻/CD28⁻ et perforine⁺,
- CD45RA⁺/CD197⁻/CD27⁻/CD28⁻ et T-bet⁺,
- CD45RA⁺/CD197⁻/CD27⁻/CD28⁻ et CD57⁺ et granzyme B⁺,
- CD45RA⁺/CD197⁻/CD27⁻/CD28⁻ et CD57⁺ et perforine⁺,
- CD45RA⁺/CD197⁻/CD27⁻/CD28⁻ et CD57⁺ et T-bet⁺,
- CD45RA⁺/CD197⁻/CD27⁻/CD28⁻ et CD57⁺ et granzyme B⁺ et perforine⁺,
- CD45RA⁺/CD197⁻/CD27⁻/CD28⁻ et CD57⁺ et granzyme B⁺ et T-bet⁺,
- CD45RA⁺/CD197⁻/CD27⁻/CD28⁻ et CD57⁺ et perforine⁺ et T-bet⁺,
- CD45RA⁺/CD197⁻/CD27⁻/CD28⁻ et CD57⁺ et granzyme B⁺ et perforine⁺ et T-bet⁺,
- CD45RA⁺/CD197⁻/CD27⁻/CD28⁻ et granzyme B⁺ et perforine⁺ ,
- CD45RA⁺/CD197⁻/CD27⁻/CD28⁻ et granzyme B⁺ et T-bet⁺,
- CD45RA⁺/CD197⁻/CD27⁻/CD28⁻ et perforine⁺ et T-bet⁺,
- CD45RA⁺/CD197⁻/CD27⁻/CD28⁻ et T-bet⁺ et granzyme B⁺ et perforine⁺.

7. Procédé *in vitro* selon la revendication 1, dans lequel le sujet est un receveur de rein à fonction de greffon pour le moment stable et sous traitement immunosuppresseur.

8. Traitement immunosuppresseur destiné à être utilisé dans un procédé pour la prévention de dysfonctionnement de greffon rénal à long terme chez un sujet ; le sujet étant un receveur de rein à fonction de greffon pour le moment stable, déterminé comme présentant un risque d'occurrence de dysfonctionnement de greffon rénal à long terme selon l'une quelconque des revendications 1 à 7.

9. Utilisation d'un kit comprenant un ou plusieurs réactifs pour la détection ou la quantification de CD45RA, CD197, CD27 et CD28 dans des lymphocytes T CD8⁺ ; dans un procédé *in vitro* pour la détermination du risque d'occurrence de dysfonctionnement de greffon rénal à long terme chez un sujet, ledit sujet étant un receveur de rein à fonction de greffon pour le moment stable.
